(19) Europäisches Patentamt
European Patent Office
Office européen des brevets

(11) **EP 2 250 959 A1**

(12) **EUROPÄISCHE PATENTANMELDUNG**

(43) Veröffentlichungstag:
**17.11.2010 Patentblatt 2010/46**

(51) Int Cl.:
*A61B 5/00* (2006.01)   *A61M 5/158* (2006.01)

(21) Anmeldenummer: **09160090.8**

(22) Anmeldetag: **13.05.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(71) Anmelder:
• **Roche Diagnostics GmbH**
  **68305 Mannheim (DE)**
  Benannte Vertragsstaaten:
  **DE**
• **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK TR**

(72) Erfinder: **Die Erfindernennung liegt noch nicht vor**

(74) Vertreter: **Stößel, Matthias et al**
  **Herzog Fiesser & Partner**
  **Bad Kreuznacher Straße 27-29**
  **68309 Mannheim (DE)**

(54) **Steuerbare Sensorinsertionsnadel**

(57) Es wird eine Insertionsvorrichtung (110) zur zumindest teilweisen Insertion einer subkutancn Vorrichtung (122) in cin Körpergewebe (124) vorgeschlagen, insbesondere eines subkutanen Sensors (126) zur Erfassung mindestens eines Analyten. Die Insertionsvorrichtung (110) weist mindestens eine Inscrtionshilfc (112) und mindestens eine subkutane Vorrichtung (122) auf. Die Insertionshilfe (112) weist mindestens einen im Wesentlichen starr ausgestalteten Grundkörper (114) zum Einführen in das Körpergewebe (124) auf, insbesondere eine Insertionsnadel (116). Die Insertionsvorrichtung (110) ist eingerichtet, um zwischen dem Grundkörper (114) und der subkutanen Vorrichtung (122) eine veränderbare Haltekraft zu erzeugen. Die Insertionsvorrichtung (110) ist eingerichtet, um während des Einführens die Haltekraft derart einzustellen, dass die subkutane Vorrichtung (122) an dem Grundkörper (114) gehalten wird. Weiterhin ist die Insertionsvorrichtung (110) eingerichtet, um nach dem Einführen die Haltekraft derart einzustellen, dass die subkutane Vorrichtung (122) von dem Grundkörper (114) lösbar ist.

Fig. 1 A

**Beschreibung**

Gebiet der Erfindung

Die Erfindung betrifft eine Insertionsvorrichtung zur Insertion einer subkutanen Vorrichtung in ein Körpergewebe sowie eine Insertionshilfe und eine subkutane Vorrichtung zum Einsatz in einer derartigen Insertionsvorrichtung. Derartige Insertionsvorrichtungen, subkutane Vorrichtungen und Insertionshilfen werden insbesondere im Bereich der medizinischen Diagnostik eingesetzt, insbesondere im Bereich des so genannten Home-Monitorings, insbesondere um eine Konzentration mindestens eines Analyten in einer Körperflüssigkeit, beispielsweise Blut oder interstitieller Flüssigkeit, zu überwachen. Auch andere Anwendungen sind jedoch möglich.

Stand der Technik

Aus dem Bereich der medizinischen Diagnostik oder Therapeutik sind subkutane Vorrichtungen bekannt, also Vorrichtungen, welche eingerichtet sind, um vollständig oder teilweise in ein Körpergewebe, beispielsweise ein interstitielles Fettgewebe, eingesetzt zu werden. Dieses Einsetzen wird auch als Insertion oder Implantation bezeichnet. Beispiele derartiger subkutaner Vorrichtungen sind insbesondere im Bereich der Diagnostik zu finden, insbesondere im Bereich der Langzeitüberwachung von Probanden, beispielsweise im Rahmen des so genannten "Home-Monitorings" oder auch im Klinikbereich. Dementsprechend kann die subkutane Vorrichtung beispielsweise mindestens einen subkutanen Sensor zur Erfassung mindestens eines Analyten in dem Körpergewebe und/oder einer Körperflüssigkeit umfassen, beispielsweise einen elektrochemischen und/oder einen optischen Sensor. Alternativ oder zusätzlich kann die subkutane Vorrichtung auch andere Arten medizinischer Vorrichtungen umfassen, wie beispielsweise Medikationsvorrichtungen, welche im Körpergewebe gezielt und vorzugsweise dosiert bestimmte Wirkstoffe einbringen können. Ohne Beschränkung weiterer möglicher Einsatzgebiete wird die Erfindung im Nachfolgenden im Wesentlichen unter Bezugnahme auf subkutane Sensoren beschrieben.

In der medizinischen Therapeutik oder Diagnostik ist es häufig erforderlich, einen oder mehrere physikalische und/oder chemische Parameter in einem Körpergewebe eines Probanden zu erfassen. Beispiele derartiger physikalischer und/oder chemischer Parameter sind Analytkonzentrationen eines oder mehrerer Analyte, wie beispielsweise Glukose. Abhängig von den erfassten Parametern kann beispielsweise eine medizinische Behandlung gewählt werden, beispielsweise eine Verabreichung bestimmter Medikamente oder eine anders gestaltete Einflusseinnahme auf den Körper oder Körperfunktionen des Probanden.

Insbesondere zum qualitativen oder quantitativen Nachweis eines oder mehrerer Analyte durch implantierbare, subkutane Sensoren sind aus dem Stand der Technik zahlreiche Beispiele bekannt. So können die subkutanen Sensoren beispielsweise auf elektrochemischen Messprinzipien basieren und eine oder mehrere chemische Substanzen (im Folgenden auch als "Testchemie" bezeichnet) umfassen, welche eine oder mehrere physikalisch und/oder chemisch messbare Eigenschaften bei Anwesenheit des mindestens einen nachzuweisenden Analyten verändern. Beispiele derartiger Testchemien sind auf Enzymen basierende Testchemien, welche beispielsweise in elektrochemischen Sensoren eingesetzt werden. Andere Messprinzipien basieren beispielsweise auf optischen Eigenschaften, bei denen mindestens eine Testchemie mindestens eine optisch nachweisbare Eigenschaft bei Anwesenheit des mindestens einen nachzuweisenden Analyten ändert. Auf sämtliche Messprinzipien kann im Rahmen der vorliegenden Erfindung Bezug genommen werden.

Eine technische und medizinische Herausforderung besteht darin, die subkutane Vorrichtung in das Körpergewebe zu implantieren (Insertion). Diese Insertion sollte möglichst schmerzfrei durchgeführt werden, und die subkutane Vorrichtung sollte derart platziert werden können, dass diese auch über einen längeren Zeitraum von beispielsweise mehreren Stunden bis hin zu mehreren Tagen zumindest teilweise in dem Körpergewebe verbleiben kann und beispielsweise Messdaten liefern kann. Aus dem Stand der Technik sind daher eine Reihe von Insertionsvorrichtungen bekannt, welche neben der subkutanen, zu implantierenden Vorrichtung eine oder mehrere Insertionshilfen umfassen. Derartige Insertionshilfen können beispielsweise ganz oder teilweise als Insertionsnadeln ausgestaltet sein, beispielsweise in Form von Kanülen, in welche die subkutane Vorrichtung eingebracht oder auf welche die subkutane Vorrichtung aufgebracht werden kann, um mit dieser in das Körpergewebe implantiert zu werden. Anschließend kann die Insertionshilfe wieder entfernt werden, wobei die subkutane Vorrichtung zumindest teilweise in dem Körpergewebe verbleibt. Ein Teil der subkutanen Vorrichtung kann dabei aus dem Körpergewebe herausragen, beispielsweise für eine spätere Entfernung der subkutanen Vorrichtung.

In vielen Fällen wird im Stand der Technik ein länglicher, subkutaner Sensor minimalinvasiv mittels einer Hohlnadel teilweise unter die Haut verbracht, beispielsweise mit einer Insertionstiefe von 10 bis 20 mm. Dies kann beispielsweise senkrecht oder unter einem Winkel erfolgen, beispielsweise 45°. Dabei wird die Haut des Probanden penetriert. Die Hohlnadel kann nach der Insertion wieder entfernt werden, und der Sensor kann in der Haut bzw. dem Gewebe verbleiben und mit einem beispielsweise extern getragenen Auswertegerät verbunden werden. Sensoren sind in der Regel in ihrer Breite und Dicke, im Vergleich zu ihrer Längsausdehnung, schmal und dadurch labil. Haut und Gewebe hindert den Sensor am Eindringen in das Körpergewebe. Um den Sensor während der Insertion zu stabilisieren, sollte dieser daher für den Vorgang gemäß dem Stand der Technik zeitweise versteift werden. Dies erfolgt bei dem beschriebenen Stand der Technik in der Regel durch die Insertionshilfe als separates Hilfsmittel, wobei die Insertionshilfe beispielsweise aus Stahl hergestellt ist, insbesondere in Form einer Nadel. Damit der Sensor auch beim Zurückziehen der Insertionsnadel in der Haut verbleibt, muss der Sensor gehalten werden. Da der Sensor in der Regel einen verdickten Halte- und/oder Sensorkontaktbereich aufweist, welcher ebenfalls aus der Insertionsnadel entfernt werden muss, ist die Insertionsnadel in der Regel geschlitzt ausgeführt. Die sich so bildende Hohlnadel kann beispielsweise ein U-Profil oder ein fast geschlossenes O-Profil oder auch ein kantiges Profil aufweisen.

Zum schmerzfreien Penetrieren der Haut und des Körpergewebes ist die Insertionsnadel in vielen Fällen am vorderen Ende mit einer Schneide versehen. Durch die Nadelform der Insertionsnadel wird während des Einstechens des Sensors gewährleistet, dass die Kraft, die das Körpergewebe auf die Sensorfront ausübt, den Sensor nicht aus der gestreckten Form bringt. Vorzugsweise ist der Nadelschlitz der Insertionsnadel dazu enger ausgestaltet als die Sensorbreite, was jedoch beim Zurückziehen und Trennen der Insertionsnadel vom Sensor eine Verjüngung im Sensorschaftbereich erfordert. Alternativ kann der Nadelschlitz seitlich an der Insertionsnadel angeordnet sein, bezogen auf die flache Sensorstruktur, wobei die Breite des Sensors die Sensordicke erheblich übersteigt. Der Halt- und Kontaktbereich kann dabei aus dem Nadelschlitz herausragen.

Eine technische Herausforderung liegt in der Freigabe des Sensors beim Zurückziehen der Insertionsnadel. Dies kann auch mittels so genannter "Peelnadeln" erfolgen. Derartige Peelnadeln können beispielsweise eine zu einem Vollrohr aufgewickelte Metallfolie umfassen, die sich beim Zurückziehen um den aus der Flucht ragenden Sensor öffnet und danach wieder verschließt.

Alternativ zur Verwendung einer Insertionsnadel kann grundsätzlich auch ein relativ flexibler Sensor ohne derartige Insertionhilfe unter die Haut verbracht werden. Eine Möglichkeit einer derartigen Insertion besteht in der Verwendung hoher Insertionsgeschwindigkeiten. Derartige Anwendungen sind jedoch grundsätzlich nachteilig, da die Insertion in der Regel mit einer hohen Unsicherheit behaftet ist und da beispielsweise eine Gefahr einer Beschädigung des Sensors bei der Insertion besteht. Zudem ist in der Regel ein mechanisch komplexer Apparat erforderlich.

Alternativ oder zusätzlich können auch Sensorelemente verwendet werden, welche lokal versteift ausgebildet sind. So beschreibt beispielsweise US 2006/0015024 A1 ein transkutanes medizinisches Element, welches einen distalen Abschnitt aufweist, welcher flexibler ausgestaltet ist als ein die Hautoberfläche penetrierender, steiferer proximaler Abschnitt. Nachteilig an derartigen Sensorelementen ist jedoch, dass auch der steifere, proximale Abschnitt nach der Insertion in dem Körpergewebe verbleibt, was Verletzungen im Körpergewebe und eine erhöhte Schmerzbelastung verursachen kann.

Im Stand der Technik sind allgemein verschiedene Arten von Insertionsvorrichtungen und Insertionshilfen im Detail beschrieben. So wird beispielsweise in EP 0 678 308 B1 ein Insertionsprinzip offenbart, bei welchem ein flexibler Fühler mittels einer hohlen Einführnadel und eines Aufsetzfußes in ein Körpergewebe implantiert werden kann. Die Einführnadel weist einen entlang einer Seite längs verlaufenden Schlitz auf, damit die Einführnadel von dem Fühler abgezogen werden kann.

Aus DE 103 06 013 A1 und US2006/0030824 ist eine temperatursensitive Kanüle zum Einbringen in ein Körpergewebe bekannt. Die Kanüle ist ein eingebrachtem Zustand biegsam und weist unterhalb eines kritischen Temperaturbereichs einen steifen Zustand und oberhalb des kritischen Temperaturbereichs einen biegsamen Zustand auf. Vor dem Einbringen der Kanüle in das Körpergewebe wird diese durch Abkühlen auf eine Temperatur unterhalb des kritischen Temperaturbereichs abgekühlt und dadurch versteift. Somit findet vor dem Einstich eine Verfestigung der Kanüle statt, während nach dem Einstich eine Erweichung des Materials stattfinden kann.

Aus DE 10 2004 002 472 B4 und US 2007/0016149 ist eine Einstechnadel für eine Einleitung eines Produkts in den menschlichen oder tierischen Körper bekannt. Die Einstechnadel weist einen distalen Nadelabschnitt und einen proximalen Nadelabschnitt mit unterschiedlichen Materialien auf, wobei der distale Nadelabschnitt eine größere Biegsamkeit als der proximale Nadelabschnitt aufweist.

Aus DE 101 17 286 A1, US 2004/0158230 und US 2008/0033399 ist eine weiche Kanüle bekannt, welche eine zunehmende Nachgiebigkeit während der Applikation aufweist. Die Kanüle weist vor der Applikation mindestens ein Material veränderlicher Härte oder mindestens zwei Materialien unterschiedlicher Härte auf, von denen das Material größerer Härte während der Applikation abgegeben wird. Das Material verändert also während der Applikation seine Härteigenschaften, was vor allem auf die Zusammensetzung der Kanüle zurückzuführen ist.

Aus WO 03/080169 A1 ist eine Insertionsnadel zum Insertieren einer subkutanen Vorrichtung bekannt, die einen Nadelkörper und einen distalen Endabschnitt aufweist. Der distale Endabschnitt weist eine Spitze auf. Weiterhin weist der Nadelkörper eine longitudinale Vertiefung auf, welche eingerichtet ist, um zumindest teilweise eine subkutane Vorrichtung aufzunehmen. Die subkutane Vorrichtung kann beispielsweise mittels eines oder mehrerer starr ausgebildeter Greifer derart auf dem Nadelkörper gehalten werden, dass diese zwar während der Insertion gehalten wird, jedoch beim Herausziehen der Insertionsnadel aus dem Körpergewebe von der Insertionsnadel heruntergeschoben werden kann.

Die bekannten Insertionsvorrichtungen und Insertionshilfen weisen grundsätzlich einige technische Nachteile auf, welche in Kauf genommen werden müssen oder auf aufwendige Weise überwunden werden müssen. So muss beispielsweise bei der Insertion mit einer Insertionsnadel der in der Regel flexible Sensorschaft durch die Nadelform der Insertionsnadel daran gehindert werden, dass dieser seitlich ausweicht. Zum Entfernen der Insertionsnadel muss der Sensor dabei in der Regel zwischen einem vorderen, distalen Teil (Sensorspitze) und einem hinteren, proximalen Teil (Kontakt- und/oder Haltestück) entweder aus der Fluchtlinie ausweichen, oder die Insertionshilfe muss mit ihrer Bewegung aus dieser Linie gebracht werden. Diese Problematik ist ausführlich beispielsweise in EP 0 678 308 B1 erläutert. Beides erfordert einen erheblichen mechanischen Aufwand, wie beispielsweise die komplizierte Gestaltung des Sensors, der Insertionsnadel und/oder der Insertionsnadel- und Sensorführung, in einem definierten Winkel oder einer Kreisbahn. Auch die oben dargestellte dynamische Insertionsmethode ist in der Regel mit einem erheblichen mechanischen Aufwand verbunden.

Ein weiterer Nachteil bekannter Insertionsvorrichtungen und Insertionshilfen besteht darin, dass Hohlnadeln in der Regel einen erheblich größeren Querschnitt aufweisen als einfache Nadeln. Dementsprechend wird die Haut mit einem erheblich größeren Querschnitt als das eigentliche Sensorprofil penetriert, was zu einem erhöhten Schmerzempfinden und zu einer erhöhten Gewebezerstörung führen kann.

Ein weiterer Nachteil bekannter Insertionsvorrichtungen und Insertionshilfen besteht in der Zuverlässigkeit der Insertion. So kann nicht in allen Fällen sichergestellt werden, dass beim Einführen der Insertionsnadel in das Körpergewebe der Sensor nicht relativ zur Insertionsnadel verrutscht. Andererseits kann beim Entfernen der Insertionsnadel aus dem Körpergewebe nicht immer zuverlässig sichergestellt werden, dass der Sensor im Körpergewebe verbleibt und nicht wieder mit der Insertionsnadel aus dem Körpergewebe entfernt wird.

Aufgabe der Erfindung

Es ist daher eine Aufgabe der vorliegenden Erfindung, eine Insertionsvorrichtung, eine Insertionshilfe und eine subkutane Vorrichtung bereitzustellen, welche die Nachteile bekannter entsprechender Vorrichtungen vermeiden. Insbesondere soll die Insertionsvorrichtung eine sichere, zuverlässige und schmerzfreie Insertion der subkutanen Vorrichtung in ein Körpergewebe ermöglichen.

Offenbarung der Erfindung

Diese Aufgabe wird durch eine Insertionsvorrichtung, eine Insertionshilfe und eine subkutane Vorrichtung mit den Merkmalen der unabhängigen Ansprüche gelöst. Die Insertionshilfe und die subkutane Vorrichtung können insbesondere für den Einsatz in einer erfindungsgemäßen Insertionsvorrichtung eingerichtet sein, so dass bezüglich möglicher Ausgestaltungen der Insertionshilfe und der subkutanen Vorrichtung auf die entsprechenden Merkmale der Insertionsvorrichtung verwiesen werden kann. Vorteilhafte Weiterbildungen der Erfindung, welche einzeln oder in Kombination realisierbar sind, sind in den Unteransprüchen dargestellt.

Es wird eine Insertionsvorrichtung zur zumindest teilweisen Insertion einer subkutanen Vorrichtung in ein Körpergewebe vorgeschlagen. Unter einer Insertion wird dabei im Rahmen der vorliegenden Erfindung ein Einbringen, beispielsweise ein Implantieren, der subkutanen Vorrichtung in das Körpergewebe verstanden. Die Begriffe "implantieren", "insertieren", "einbringen", "einführen" und "einfügen" werden dabei im Folgenden im wesentlichen synonym verwendet und beinhalten ein zumindest teilweises Verbringen der subkutanen Vorrichtung in das Körpergewebe. Die subkutane Vorrichtung kann vollständig oder teilweise in das Körpergewebe eingebracht werden, wobei optional ein Teil der subkutanen Vorrichtung aus dem Körpergewebe herausragen kann, beispielsweise um mit einer Messvorrichtung und/oder einem Ansteuer- und/oder Auswertegerät verbunden zu werden. Unter einer subkutanen Vorrichtung ist allgemein eine Vorrichtung zu verstehen, welche in ein Körpergewebe implantierbar ist. Der Begriff "subkutan" wird dabei gleichbedeutend mit dem in der Literatur ebenfalls häufig verwendeten Begriff "transkutan" eingesetzt, da in beiden Fällen eine Insertion insbesondere durch eine Haut eines Probanden hindurch in das Körpergewebe hinein erfolgen kann. Auch eine andere Art der Insertion, ohne Penetrierung der Haut, ist jedoch grundsätzlich möglich, beispielsweise in ein bereits offen liegendes Körpergewebe (beispielsweise bei einer Operation) und/oder durch ein nicht von einer Haut im eigentlichen Sinne und/oder im Sinne einer Epidermis bedecktes Körpergewebe. Allgemein kann der Begriff der Haut jedoch weit gefasst sein und kann beispielsweise eine Epidermis, eine Dermis, eine Subcutis oder eine andere Art von Haut umfassen. Die Insertion kann dabei vollständig durch die Haut hindurch erfolgen, oder die Haut kann selbst bereits teilweise als Körpergewebe verstanden werden, so dass die Insertion beispielsweise lediglich durch eine oder mehrere oberste Hautschichten hindurch erfolgen kann.

Die subkutane Vorrichtung kann insbesondere mindestens eine medizinische Funktion aufweisen, insbesondere mindestens eine diagnostische und/oder mindestens eine therapeutische Funktion. Dementsprechend kann die subkutane Vorrichtung beispielsweise als subkutaner Sensor ausgestaltet sein oder mindestens einen derartigen subkutanen Sensor umfassen, wobei der subkutane Sensor eingerichtet ist, um mindestens einen Analyten qualitativ oder quantitativ zu erfassen. Beispielsweise kann der Sensor zum qualitativen und/oder quantitativen Nachweis mindestens eines Analyten in dem Körpergewebe selbst und/oder in einem oder mehreren in dem Körpergewebe enthaltender Körperflüssigkeiten, wie beispielsweise Blut und/oder interstitieller Flüssigkeit, ausgestaltet sein. Bei dem Analyten kann es sich insbesondere um mindestens einen Metaboliten handeln. Alternativ oder zusätzlich zum Nachweis mindestens eines Analyten kann der Sensor auch grundsätzlich eingerichtet sein, um mindestens eine physikalische und/oder chemische Eigenschaft des Körpergewebes und/oder des Probanden zu erfassen, wie beispielsweise eine Temperatur, einen Blutdruck oder Ähnliches. Die Erfindung wird im Folgenden im Wesentlichen unter Bezugnahme auf Glukosesensoren beschrieben, wobei jedoch auch andere Ausgestaltungen alternativ oder zusätzlich möglich sind. Der subkutane Sensor kann, wie oben dargestellt, beispielsweise grundsätzlich entsprechend dem Stand der Technik ausgestaltet sein und kann beispielsweise mindestens eine Testchemie zur Erfassung des mindestens einen Analyten aufweisen. Beispielsweise kann eine Testchemie enthalten sein, welche bei Anwesenheit des mindestens einen Analyten mindestens eine physikalisch und/oder chemisch nachweisbare Eigenschaft ändert, beispielsweise eine elektrochemisch messbare Eigenschaft und/oder eine optisch messbare Eigenschaft. Dementsprechend kann der subkutane Sensor beispielsweise als elektrochemischer Sensor ausgestaltet sein. Bezüglich möglicher Testchemien kann auf den Stand der Technik verwiesen werden. Der subkutane Sensor kann dementsprechend beispielsweise zwei oder drei oder mehrere Elektroden umfassen, mittels derer elektrochemisch beispielsweise der mindestens eine Analyt quantitativ und/oder qualitativ nachgewiesen werden kann. Diese mindestens zwei, vorzugsweise drei oder mehr Elektroden können beispielsweise mittels entsprechender elektrischer Kontakte, vorzugsweise außerhalb des Körpergewebes, kontaktiert werden und beispielsweise mit der oben beschriebenen Messvorrichtung und/oder der Ansteuer- und/oder Auswertevorrichtung verbunden werden.

Alternativ oder zusätzlich kann die subkutane Vorrichtung auch andere Vorrichtungen umfassen. Beispielsweise kann die subkutane Vorrichtung mindestens eine therapeutische Funktion umfassen und entsprechend eingerichtet sein, um diese therapeutische Funktion durchzuführen oder zumindest zu unterstützen. Beispielsweise kann zu diesem Zweck die subkutane Vorrichtung mindestens einen Aktor umfassen, welcher direkt oder indirekt auf das Körpergewebe und/oder eine Körperflüssigkeit und/oder den Probanden einwirkt, beispielsweise einen elektrischen und/oder mechanischen und/oder thermischen Aktor. Wiederum alternativ oder zusätzlich kann der Aktor auch ausgestaltet sein, um eine Medikationsfunktion auszuüben. So kann beispielsweise mittels der subkutanen Vorrichtung mindestens ein Medikament und/oder eine anders gestaltete Substanz an das Körpergewebe und/oder den Probanden abgegeben werden. Verschiedene Ausgestaltungen oder Kombinationen sind möglich, beispielsweise subkutane Vorrichtungen, welche diagnostische und therapeutische Funktionen kombinieren. Im Folgenden wird im Wesentlichen Bezug genommen auf subkutane Vorrichtungen in Form streifenförmiger subkutaner Sensoren zum Nachweis mindestens eines Analyten, beispielsweise streifenförmige, subkutane elektrochemische Sensoren. Auch andere Ausgestaltungen sind jedoch grundsätzlich möglich, beispielsweise drahtförmige Ausgestaltungen.

Die Insertionsvorrichtung umfasst mindestens eine derartige subkutane Vorrichtung, welche in das Körpergewebe implantiert bzw. insertiert werden kann. Weiterhin umfasst die Insertionsvorrichtung mindestens eine Insertionshilfe, mittels derer die subkutane Vorrichtung insertiert werden kann. Unter einer Insertionshilfe ist dabei eine Vorrichtung zu verstehen, welche dem Zweck der Insertion der subkutanen Vorrichtung dient und welche vorzugsweise nach der Insertion wieder aus dem Körpergewebe entfernt werden kann. Vorzugsweise kann das Entfernen der Insertionshilfe unmittelbar nach Einbringung der subkutanen Vorrichtung in das Körpergewebe erfolgen. Die Insertionshilfe ist vorzugsweise derartig eingerichtet, dass diese lediglich vorübergehend im Körpergewebe verbleibt, beispielsweise für eine Zeitdauer von wenigen Millisekunden bis einige Sekunden, beispielsweise maximal 60 Sekunden, vorzugsweise maximal 30 Sekunden und besonders bevorzugt maximal 10 Sekunden oder weniger. Im Gegensatz dazu kann die subkutane Vorrichtung über mehrere Minuten, vorzugsweise mindestens mehrere Stunden und besonders bevorzugt sogar mehrere Tage oder sogar Wochen in dem Körpergewebe verbleiben.

Die Insertionshilfe umfasst mindestens einen im Wesentlichen starren Grundkörper zum Einführen in das Körpergewebe. Beispielsweise kann der im Wesentlichen starre Grundkörper eine Nadelform aufweisen, also eine langgestreckte Form mit einer Längserstreckung, welche die lateralen Dimensionen des starren Grundkörpers erheblich übersteigt, beispielsweise um einen Faktor 10, vorzugsweise um mindestens einen Faktor 100 oder mehr. Der starre Grundkörper ist vorzugsweise einteilig ausgestaltet, kann jedoch grundsätzlich auch mehrteilig ausgebildet sein. Der starre Grundkörper kann, wie unten noch näher ausgeführt wird, vorzugsweise mindestens eine Einführspitze aufweisen, mittels derer die Insertionshilfe in das Körpergewebe einbringbar ist. Die Einführspitze kann beispielsweise eine Hautoberfläche des Probanden und/oder das Körpergewebe penetrieren. Die Einführspitze kann beispielsweise als geschliffene, scharfkantige Einführspitze oder auch als runde Einführspitze ausgestaltet sein. Besonders bevorzugt sind atraumatische Einführspitzen. Unter einem im Wesentlichen starren Grundkörper ist dabei ein Körper oder allgemein Element zu verstehen, welcher bzw. welches seine äußere Gestalt bei üblichen, beim Insertionsvorgang auftretenden Kräften nicht oder nur unwesentlich verändert. Insbesondere soll der starre Grundkörper seine Gestalt unter Einwirkung seiner eigenen Gewichtskraft nicht oder nur unwesentlich verändern. Der starre Grundkörper kann dementsprechend beispielsweise aus einem starren Material hergestellt sein, vorzugsweise einem biologisch verträglichen Material, beispielsweise einem metallischen Material, vorzugsweise Edelstahl, und/oder einem keramischen Material und/oder einem starren Kunststoff. Auch Kombinationen verschiedener Materialien sind möglich. Der starre Grundkörper wird im Folgenden auch und ohne Beschränkung weiterer möglicher Ausgestaltungen als Insertionsnadel bezeichnet.

Die Insertionsvorrichtung ist eingerichtet, um zwischen dem Grundkörper der Insertionshilfe und der subkutanen Vorrichtung, beispielsweise zwischen der Insertionsnadel und dem subkutanen Sensor, eine veränderbare Haltekraft zu erzeugen. Unter einer veränderbaren Haltekraft ist dabei eine Kraft zu verstehen, welche zwischen der subkutanen Vorrichtung und dem Grundkörper wirkt und hinsichtlich ihres Betrags und/oder ihrer Richtung veränderbar ist. Insbesondere kann diese durch die Insertionsvorrichtung selbst veränderbar sein. Insbesondere kann die Haltekraft eine Kraftkomponente senkrecht zu einer Verbindungslinie zwischen dem Grundkörper und der subkutanen Vorrichtung aufweisen. Beispielsweise können der Grundkörper und die subkutane Vorrichtung zumindest teilweise eine längliche Gestalt aufweisen, mit Längserstreckungsachsen, welche beispielsweise parallel angeordnet sind. In diesem Fall kann die veränderbare Haltekraft beispielsweise eine Komponente senkrecht zu diesen Längserstreckungsachsen aufweisen.

Die Insertionsvorrichtung ist eingerichtet, um während des Einführens und nach dem Einführen unterschiedliche Haltekräfte einzustellen. So ist die Insertionsvorrichtung derart eingerichtet, dass während des Einführens die Haltekraft derart eingestellt wird, dass die subkutane Vorrichtung an dem Grundkörper gehalten wird. Weiterhin ist die Insertionsvorrichtung eingerichtet, um nach dem Einführen die Haltekraft derart einzustellen, dass die subkutane Vorrichtung von dem Grundkörper lösbar ist. Dieser Wechsel von mindestens einer ersten, während des Einführens wirkenden Haltekraft zu mindestens einer zweiten, von der ersten Haltekraft verschiedenen und nach dem Einführen wirkenden Haltekraft kann beispielsweise automatisch durch die Insertionsvorrichtung erfolgen. Zu diesem Zweck kann die Insertionsvorrichtung beispielsweise eine Steuerung aufweisen, welche diesen Wechsel von der ersten Haltekraft zur zweiten Haltekraft, beispielsweise kontinuierlich, schrittweise oder stufenweise, vornehmen kann. Die Steuerung kann beispielsweise eine elektronische Steuerung und/oder eine Steuerung durch eine Datenverarbeitungsvorrichtung umfassen und kann beispielsweise ganz oder teilweise in der Insertionshilfe und/oder einem anderen Teil der Insertionsvorrichtung integriert sein. Beispielsweise kann diese Steuerung in einem wiederverwertbaren Teil der Insertionsvorrichtung (reusable) aufgenommen sein, wohingegen die transkutane Vorrichtung ganz oder teilweise beispielsweise als Einwegteil (disposable) ausgestaltet sein kann oder zumindest ein derartiges Einwegteil umfassen kann. Auch die transkutane Vorrichtung kann jedoch ein oder mehrere wiederverwertbare Teile umfassen. Alternativ oder zusätzlich zur Vornahme des Wechsels von der ersten Haltekraft zur zweiten Haltekraft kann der Wechsel von der ersten Haltekraft zur zweiten Haltekraft auch beispielsweise durch einen Benutzer ausgelöst werden, beispielsweise durch eine Betätigung mindestens eines Betätigungselements, beispielsweise eines Auslöseknopfes, und/oder durch ein einfaches Herausziehen der Insertionshilfe aus dem Körpergewebe.

Unter "von dem Grundkörper lösbar" ist dabei ein Zustand zu verstehen, bei welchem die Haltekraft nicht mehr oder lediglich in verringertem Maße die subkutane Vorrichtung an dem Grundkörper hält. Zu diesem Zweck kann die Haltekraft beispielsweise von ihrem Betrag und/oder ihrer Richtung her derart ausgestaltet sein, dass diese beispielsweise durch Reibungskräfte an dem Körpergewebe leicht überwunden werden kann, so dass beim Herausziehen der Insertionshilfe aus dem Körpergewebe die subkutane Vorrichtung in dem Körpergewebe zumindest teilweise verbleibt. Alternativ kann die Haltekraft auch vollständig auf Null gesetzt werden. Wiederum alternativ kann die Haltekraft auch derart ausgestaltet werden, dass die subkutane Vorrichtung von dem Grundkörper abgestoßen wird.

Die Haltekraft kann hinsichtlich ihres Betrages und/oder ihrer Richtung insbesondere derart eingerichtet sein, dass diese während des Einführens eine räumliche Verlagerung zwischen der subkutanen Vorrichtung und dem Grundkörper in einer Insertionsrichtung zumindest weitgehend verhindert. Sind beispielsweise während des Einführens der Grundkörper und die subkutane Vorrichtung bzw. der eingeführte Teil der subkutanen Vorrichtung im Wesentlichen parallel zueinander orientiert, beispielsweise indem deren Längserstreckungsachsen um einen Winkel von nicht mehr als 10°, vorzugsweise um nicht mehr als 5° voneinander abweichen, so kann beispielsweise die Haltekraft während des Einführens derart ausgestaltet sein, dass diese einer räumlichen Verlagerung des Grundkörpers und der subkutanen Vorrichtung relativ zueinander in einer Richtung parallel zu deren Längserstreckungsachsen zumindest entgegenwirkt. Damit steht die vorgeschlagene Insertionsvorrichtung im Unterschied beispielsweise zu den in WO 03/080169 offenbarten Greifern, welche noch eine Verschiebung parallel zu den Längserstreckungsachsen ermöglichen. Zudem weisen diese Greifer keine zwei unterschiedlichen Haltekräfte während des Einführens und nach dem Einführen auf.

Der Wechsel, das heißt die Veränderung der Haltekräfte während des Einführens und nach dem Einführen kann kontinuierlich oder auch beispielsweise stufenweise oder diskontinuierlich erfolgen. Auch mehr als zwei unterschiedliche Haltekräfte während unterschiedlicher Phasen des Einführens und nach dem Einführen können vorgesehen sein. Die Haltekraft muss dabei nicht notwendigerweise monoton in einer Richtung geändert werden, sondern kann beispielsweise auch nicht-monotone Veränderungen umfassen. Die Haltekraft kann insbesondere eine nicht-mechanische Haltekraft umfassen, also keine Haltekraft, welche beispielsweise durch einen Formschluss oder einen Kraftschluss hervorgerufen wird. Verschiedene Möglichkeiten zur Erzeugung einer derartigen veränderbaren Haltekraft sind grundsätzlich aus dem Stand der Technik bekannt und auch im Rahmen der vorliegenden Insertionsvorrichtung einsetzbar, auch in grundsätzlich beliebiger Kombination.

In einer ersten Ausführungsform der Erfindung umfasst die Haltekraft eine elektrostatische Haltekraft. Derartige elektrostatische Haltekräfte sind beispielsweise mittels zweier oder mehrerer Elektroden, welche mit entsprechenden Potenzialen beaufschlagt werden können, leicht zu erzeugen und zu verändern. Zur Einstellung bzw. zur Veränderung der Haltekraft ist dementsprechend lediglich eine Veränderung eines oder mehrerer der Potenziale der Elektroden erforderlich. Dementsprechend kann der Grundkörper beispielsweise mindestens eine erste Elektrode aufweisen, wobei die subkutane Vorrichtung mindestens eine zweite Elektrode aufweist. Auch eine Ausgestaltung mit mehr als einer ersten Elektrode und/oder mit mehr als einer zweiten Elektrode ist grundsätzlich möglich. Die erste Elektrode und/oder die zweite Elektrode können als separate Elektroden in dem Grundkörper bzw. der subkutanen Vorrichtung vorgesehen sein, können jedoch auch großflächig ausgestaltet sein und beispielsweise größere Bestandteile des Grundkörpers bzw. der subkutanen Vorrichtung bilden. Insbesondere kann beispielsweise der gesamte Grundkörper als erste Elektrode ausgestaltet sein, beispielsweise indem dieser vollständig als metallischer Grundkörper ausgestaltet wird, welcher mit einem entsprechenden Potenzial beaufschlagt werden kann. Allgemein kann die Insertionsvorrichtung eingerichtet sein, um die erste Elektrode und die zweite Elektrode mit unterschiedlichen elektrischen Potenzialen zu beaufschlagen. Dementsprechend bildet sich zwischen der ersten Elektrode und der zweiten Elektrode mindestens eine Potenzialdifferenz, also eine Spannung, aus. Aufgrund dieser elektrischen Potenzialdifferenz wirkt eine elektrostatische Kraft zwischen der ersten Elektrode und der zweiten Elektrode, welche eine Anziehung zwischen dem Grundkörper und der subkutanen Vorrichtung oder eine Abstoßung zwischen dem Grundkörper und der subkutanen Vorrichtung bewirken kann. Der Betrag und die Richtung dieser elektrostatischen Kraft sind beispielsweise durch die Elektrodengeometrien und/oder gegebenenfalls durch zwischen den Elektroden vorhandenen Materialien und/oder durch die Potenzialdifferenz bedingt und entsprechend veränderbar. Die Insertionsvorrichtung kann beispielsweise mindestens eine Spannungsquelle aufweisen, welche die unterschiedlichen elektrischen Potenziale generieren kann bzw. die Potenzialdifferenz zwischen der ersten Elektrode und der zweiten Elektrode. Um unterschiedliche Haltekräfte zu erzeugen, können beispielsweise der Betrag und/oder die Richtung dieser Potenzialdifferenz verändert werden.

Zwischen der ersten Elektrode und der zweiten Elektrode können dabei grundsätzlich ein oder mehrere Isolatoren angeordnet sein. Dieser mindestens eine Isolator beeinflusst beispielsweise über seine Dielektrizitätskonstante die Haltekraft und kann beispielsweise mindestens ein dielektrisches Material umfassen. Der mindestens eine Isolator kann beispielsweise Bestandteil der Insertionshilfe und/oder auch Bestandteil der subkutanen Vorrichtung sein. Beispielsweise kann der mindestens eine Isolator mindestens einen keramischen Isolator und/oder mindestens einen Kunststoffisolator umfassen. Mittels des mindestens einen Isolators kann beispielsweise ein direkter Kontakt zwischen der ersten Elektrode und der zweiten Elektrode verhindert werden, was zu einem Kurzschluss führen könnte. Weiterhin können Richtung und/oder Betrag der Haltekraft durch den mindestens einen Isolator beeinflusst werden.

Alternativ oder zusätzlich zur Verwendung einer elektrostatischen Haltekraft kann die Haltekraft grundsätzlich auch eine magnetische Haltekraft umfassen. Eine derartige magnetische Haltekraft, welche als veränderbare Haltekraft ausgestaltet sein sollte, kann beispielsweise durch Verwendung mindestens eines Permanentmagneten und/oder mindestens eines Elektromagneten oder einer Kombination derartiger Magnete erzeugt werden. Derartige veränderbare magnetische Haltekräfte sind beispielsweise aus Magnetschaltern bekannt. Die Veränderung der magnetischen Haltekraft kann beispielsweise bei Elektromagneten durch Veränderung eines Stroms erfolgen, beispielsweise durch eine Veränderung in einer Stromrichtung und/oder eine Veränderung in einem Betrag des Stroms. Alternativ oder zusätzlich kann die Veränderung der magnetischen Haltekraft beispielsweise bei Permanentmagneten insbesondere durch eine mechanische Verlagerung des mindestens einen Permanentmagneten erfolgen. Derartige Prinzipien, bei welchem mittels Permanentmagneten geschaltet wird, sind in Form von Magnetschaltern aus dem Stand der Technik grundsätzlich bekannt. Auch eine Kombination von einem oder mehreren Permanentmagneten mit einem oder mehreren Elektromagneten ist denkbar. Besonders bevorzugt ist aufgrund der einfachen Steuerung die Verwendung mindestens eines elektromagnetischen Halteelements. Dementsprechend kann die Insertionshilfe und/oder die subkutane Vorrichtung mindestens ein elektromagnetisches Halteelement umfassen, welches zur Erzeugung eines magnetischen Feldes eingerichtet ist. Das elektromagnetische Halteelement kann beispielsweise mindestens eine Leiterbahnschleife und/oder eine Spule umfassen. Dabei kann das elektromagnetische Halteelement beispielsweise auf oder in der Insertionshilfe angeordnet sein oder, alternativ oder zusätzlich, in oder auf der subkutanen Vorrichtung. Ist lediglich eines der beiden Elemente, also entweder die Insertionshilfe oder die subkutane Vorrichtung, mit einem elektromagnetischen Halteelement zur Erzeugung eines magnetischen Feldes ausgestaltet, so kann das jeweils andere Element, also die subkutane Vorrichtung oder die Insertionshilfe, ein entsprechendes Gegenelement umfassen, beispielsweise ebenfalls ein magnetisches Halteelement (beispielsweise ein permanentmagnetisches Halteelement und/oder ein elektromagnetisches Halteelement) oder ein passives Element, wie beispielsweise ein weichmagnetisches Element und/oder ein ferromagnetisches Element, welches mit dem elektromagnetischen Halteelement zusammenwirken kann. Ist mindestens ein elektromagnetisches Halteelement vorgesehen, vorzugsweise in der Insertionshilfe, so kann die Insertionsvorrichtung beispielsweise mindestens eine Stromquelle zur Erzeugung eines veränderbaren Stroms aufweisen, welcher in das mindestens eine elektromagnetische Halteelement eingespeist werden kann. Diese Stromquelle kann beispielsweise als veränderbare Stromquelle ausgestaltet sein, so dass der Strom hinsichtlich seines Betrags und/oder seiner Richtung einstellbar ist.

Wiederum alternativ oder zusätzlich zu den oben beschriebenen elektrostatischen und/oder magnetischen Prinzipien zur Erzeugung der veränderbaren Haltekraft lassen sich weitere Prinzipien zur Erzeugung der veränderbaren Haltekraft einsetzen. Beispielsweise lassen sich Adhäsionskräfte zwischen dem Grundkörper und der subkutanen Vorrichtung gezielt beeinflussen, beispielsweise wiederum durch elektrische Felder und/oder auf anderem elektrischen und/oder magnetischen Wege. Insbesondere kann die Insertionsvorrichtung eingerichtet sein, um eine elektrische Ladungsträgerdichte und/oder eine elektrische Leitfähigkeit im Bereich mindestens einer Haftoberfläche der Insertionshilfe und/oder der subkutanen Vorrichtung zu beeinflussen. Unter einer Haftoberfläche ist dabei eine Oberfläche der Insertionshilfe und/oder der subkutanen Vorrichtung zu verstehen, welche dem jeweils anderen Element benachbart angeordnet ist, beispielsweise indem die subkutane Vorrichtung auf der Haftoberfläche der Insertionshilfe aufliegt oder umgekehrt. Die Haftoberfläche kann somit Bestandteil der Insertionshilfe oder Bestandteil der subkutanen Vorrichtung sein. Alternativ können die Insertionshilfe und die subkutane Vorrichtung derartige Haftoberflächen aufweisen. Beispielsweise kann zwischen der subkutanen Vorrichtung und der Insertionshilfe mindestens eine Adhäsionskraft bestehen, welche beispielsweise durch adhäsive Materialien begünstigt und/oder hervorgerufen werden kann, wie beispielsweise Klebstoffe und/oder andere Materialien, beispielsweise adhäsive organische Materialien. Weiterhin sind hier, alternativ oder zusätzlich zu den genannten Materialien, exemplarisch als adhäsive Materialien Zucker oder Salze, beispielsweise in Form von Zuckerlösungen und/oder Salzlösungen, zu nennen, welche beispielsweise bei Veränderung des Wassergehalts ihrer Umgebung bei der Insertion gelöst werden, beispielsweise automatisch. In diesen oder auch in anderen Fällen kann die Adhäsionskraft zwischen der subkutanen Vorrichtung und der Insertionshilfe gezielt dadurch beeinflusst werden, dass die elektrische Ladungsträgerdichte und/oder die elektrische Leitfähigkeit im Bereich der mindestens einen Haftoberfläche der Insertionshilfe und/oder der subkutanen Vorrichtung beeinflusst wird. Dies kann beispielsweise unter Verwendung eines Feldeffekts erfolgen. Mittels eines Feldeffekts, der beispielsweise bei einem Feldeffekttransistor ausgenutzt wird, kann, beispielsweise durch Veränderung einer Spannung an mindestens einer Elektrode, beispielsweise einer Schaltelektrode oder so genannten Gate-Elektrode, die Ladungsträgerdichte und/oder die elektrische Leitfähigkeit im Bereich der mindestens einen Haftoberfläche beeinflusst werden. Die mindestens eine Haftoberfläche kann dementsprechend beispielsweise ein halbleitendes Material aufweisen, beispielsweise ein organisches und/oder ein anorganisches halbleitendes Material. Dementsprechend können beispielsweise in organischen Feldeffekttransistoren einsetzbare Materialien zumindest im Bereich der Haftoberfläche eingesetzt werden. Dies können beispielsweise leitfähige oder halbleitende organische Materialien sein, insbesondere konjugierte Polymere oder andere konjugierte organische Materialien, also Materialien mit einem konjugierten π-Elektronensystem. Beispiele derartiger organischer leitfähiger oder halbleitender Materialien sind Polyacetylen, Polyanilin, Polytiophen, Polyparaphenylen oder andere organische Materialien mit ausgedehnten π-Elektronensystemen. Die mindestens eine Haftoberfläche kann dementsprechend mindestens einen Feldeffekttransistor, beispielsweise einen MOSFET, umfassen, beispielsweise einen organischen Feldeffekttransistor, wobei das Halbleitermaterial des Feldeffekttransistors jeweils dem anderen Element zuweist. Ist die Haftoberfläche beispielsweise Bestandteil der Insertionshilfe, so soll das halbleitende Material der subkutanen Vorrichtung zuweisen bzw. deren korrespondierender Haftoberfläche. Ist der Feldeffekttransistor Bestandteil der subkutanen Vorrichtung, so soll dessen Halbleitermaterial der Insertionshilfe zuweisen. Auch Kombinationen mehrerer Feldeffekttransistoren sind möglich.

Die genannten Beispiele zur Erzeugung einer veränderbaren Haltekraft, welche auch in Kombination einsetzbar sind, sind lediglich mögliche Beispiele, wie eine derartige veränderbare Haltekraft erzeugbar und veränderbar ist. Aus dem Stand der Technik sind grundsätzlich verschiedene andere Möglichkeiten bekannt, um eine Kraft zwischen der Insertionshilfe und der subkutanen Vorrichtung zu erzeugen, welche vorzugsweise nichtmechanisch ist, welche vorzugsweise berührungsfrei funktioniert und welche veränderbar ist.

Die Insertionshilfe kann insbesondere eine Auflagefläche zum Anlegen der subkutanen Vorrichtung während des Einführens aufweisen. Diese Auflagefläche kann beispielsweise die oben beschriebene mindestens eine optionale Haftoberfläche der Insertionshilfe umfassen bzw. bilden. Insbesondere kann die Auflagefläche als im Wesentlichen ebene Auflagefläche ausgestaltet sein, im Unterschied beispielsweise zu bekannten Hohlnadeln zum Einführen eines subkutanen Sensors. Insbesondere kann die Insertionshilfe derart ausgestaltet sein, dass diese die subkutane Vorrichtung nicht umschließt, so dass die subkutane Vorrichtung ohne die Haltekraft senkrecht zur Längserstreckungsrichtung der Insertionshilfe frei von der Insertionshilfe entfernbar sein kann.

Die Insertionshilfe kann auf einer der Auflagefläche gegenüberliegenden Seite ein Profil aufweisen, insbesondere eines der folgenden Profile: ein polygonales Profil, insbesondere ein Dreiecksprofil oder ein Trapezprofil; ein rundes Profil, insbesondere ein kreissegmentförmiges Profil. Eine Profilierung, beispielsweise in der beschriebenen Art, kann die Stabilität der Insertionshilfe beim Einführen in das Körpergewebe erhöhen, insbesondere wenn diese zumindest teilweise als Insertionsnadel ausgestaltet ist. Gleichzeitig kann eine Profilreduktion des Stichkanals erfolgen, und das Schmerzempfinden kann reduziert werden. Beispielsweise kann die Insertionsnadel mindestens eine Einführspitze aufweisen. Unter einer Einführspitze ist dabei ein scharfkantiges oder spitzes Element zu verstehen, wobei sowohl geschliffene Spitzen als auch Spitzen mit rundem Querschnitt zum Einsatz kommen können, insbesondere atraumatische Einführspitzen. Besonders bevorzugt ist es, wenn die Insertionshilfe im Bereich der Einführspitze mindestens eine Erhöhung aufweist, wobei die Erhöhung beim Einführen die subkutane Vorrichtung zumindest teilweise überdeckt, so dass beim Einführen der Insertionshilfe mit der subkutanen Vorrichtung eine Kraftwirkung auf die subkutane Vorrichtung zumindest reduziert wird. Auf der der subkutanen Vorrichtung zuweisenden Seite kann die Erhöhung eine Rampe aufweisen, so dass beim Entfernen der Insertionshilfe aus dem Körpergewebe die subkutane Vorrichtung leichter von der Insertionshilfe entfernt werden kann, beispielsweise indem diese über die genannte Rampe gleitet. Beispielsweise kann sich die Erhöhung unmittelbar oder über die Rampe an die Auflagefläche anschließen, beispielsweise die im Wesentlichen ebene Auflagefläche, welche beispielsweise eine Abflachung in einer ansonsten beispielsweise runden Insertionsnadel beinhalten kann.

Wie oben dargestellt, umfasst die Insertionsvorrichtung also mindestens eine subkutane Vorrichtung und mindestens eine Insertionshilfe, wobei zwischen diesen Elementen eine veränderbare Haltekraft erzeugbar ist. Neben der Insertionsvorrichtung in einer oder mehreren der oben beschriebenen Ausgestaltungen werden daher weiterhin eine Insertionshilfe und eine subkutane Vorrichtung vorgeschlagen, welche insbesondere eingerichtet sein können, um in der Insertionsvorrichtung gemäß einer oder mehrerer der oben beschriebenen Ausführungsformen eingesetzt zu werden. Dementsprechend kann zumindest weitgehend auf die obige Beschreibung verwiesen werden. Auch in anderen Insertionsvorrichtungen sind die Insertionshilfe und die subkutane Vorrichtung jedoch grundsätzlich einsetzbar, insbesondere solange das beschriebene Prinzip der Erzeugung einer veränderbaren Haltekraft realisiert wird.

Die vorgeschlagene Insertionshilfe dient, wie oben dargestellt, zur zumindest teilweisen Insertion einer subkutanen Vorrichtung in ein Körpergewebe. Die Insertionshilfe weist mindestens einen im Wesentlichen starr ausgebildeten Grundkörper zum Einführen in das Körpergewebe auf, insbesondere mindestens eine Insertionsnadel. Diese Insertionsnadel kann beispielsweise als Vollnadel ausgestaltet sein, also ohne Hohlraum. Die Insertionsnadel kann, zur optionalen Erzeugung einer Auflagefläche gemäß der obigen Beschreibung, beispielsweise eine Abflachung umfassen. Die Insertionshilfe ist eingerichtet, um mit der subkutanen Vorrichtung derart zusammenzuwirken, dass zwischen dem Grundkörper und der subkutanen Vorrichtung eine veränderbare Haltekraft erzeugbar ist. Zu diesem Zweck kann die Insertionshilfe beispielsweise mindestens ein erstes Halteelement umfassen. Die subkutane Vorrichtung kann dementsprechend beispielsweise mindestens ein zweites Halteelement umfassen und/oder ausgestaltet sein, um auf andere Weise mit dem ersten Halteelement zusammenzuwirken. Wie oben dargestellt, kann es sich bei dem mindestens einen ersten Halteelement der Insertionshilfe beispielsweise um ein elektrostatisches und/oder ein magnetisches, insbesondere ein elektromagnetisches, und/oder um ein auf einer Veränderung einer Ladungsträgerdichte und/oder einer Leitfähigkeit im Bereich einer Haftoberfläche der Insertionshilfe basierendes erstes Halteelement handeln. Auch Kombinationen sind möglich. Für weitere Ausgestaltungen kann auf die obige Beschreibung verwiesen werden. Das Halteelement kann beispielsweise über entsprechende Zuleitungen in der Insertionshilfe mit einer Steuerung verbunden werden, so dass die Haltekraft gezielt verändert werden kann. Für weitere mögliche Ausgestaltungen kann auf die obige Beschreibung verwiesen werden, dort insbesondere auf Merkmale, welche die Insertionshilfe betreffen.

Die vorgeschlagene subkutane Vorrichtung ist für eine Insertion in ein Körpergewebe geeignet, wobei die subkutane Vorrichtung vollständig oder teilweise in das Körpergewebe eingeführt werden kann, beispielsweise transkutan. Insbesondere kann die subkutane Vorrichtung einen implantierbaren Teil aufweisen und einen nicht-implantierbaren Teil. Insbesondere kann die subkutane Vorrichtung ganz oder teilweise als subkutaner Sensor ausgestaltet sein oder einen subkutanen Sensor umfassen, insbesondere einen subkutanen Sensor zur Erfassung mindestens eines Analyten, beispielsweise in dem Körpergewebe und/oder in Körperflüssigkeit. Wie oben dargestellt, sind jedoch auch andere Ausgestaltungen der subkutanen Vorrichtung möglich, beispielsweise Ausgestaltungen, bei denen der Sensor mindestens eine andere physikalische und/oder chemische Eigenschaft eines Probanden erfasst und/oder Ausgestaltungen, bei welchen die subkutane Vorrichtung mindestens eine Medikationsvorrichtung und/oder mindestens einen Aktor zur Einwirkung auf das Körpergewebe und/oder den Probanden umfasst. Die subkutane Vorrichtung kann, wie oben dargestellt, mindestens einen implantierbaren Teil umfassen. Dieser implantierbare Teil ist vorzugsweise vollständig oder teilweise flexibel ausgestaltet, so dass sich dieser bei üblichen Bewegungen innerhalb des Körpergewebes verformen kann. Dementsprechend kann der subkutane Sensor beispielsweise einen flexiblen Träger aufweisen, beispielsweise einen streifenförmigen flexiblen Träger und/oder einen rohrförmigen flexiblen Träger. Der Träger kann beispielsweise als flexibles Material ein Kunststoffmaterial und/oder ein Papiermaterial und/oder ein keramisches Material umfassen, wobei auch Laminatmaterialien möglich sind. Der Träger kann eine oder mehrere Funktionselemente aufnehmen. Beispielsweise können, wie oben dargestellt, ein oder mehrere Testfelder und/oder Elektroden eines subkutanen Sensors auf dem Träger und/oder in dem Träger aufgenommen sein, beispielsweise zur elektrochemischen und/oder optischen Erfassung des mindestens einen Analyten in der Körperflüssigkeit bzw. dem Körpergewebe. Diesbezüglich kann auf übliche Testelemente aus dem Bereich der Diagnostik verwiesen werden, welche beispielsweise zum Nachweis mindestens eines Analyten in einer Körperflüssigkeit eingesetzt werden können, sowie auf den oben zitierten Stand der Technik. Weiterhin kann die subkutane Vorrichtung beispielsweise eine oder mehrere Zuleitungen umfassen, welche beispielsweise über den Träger in einen Bereich außerhalb des Körpergewebes geführt werden können, um dort beispielsweise mit einer Steuerung verbunden zu werden. Beispielsweise kann der Träger als Einwegeinheit (disposable) ausgestaltet sein und beispielsweise mit einer Mehrwegeinheit (reusable) verbunden werden.

Die subkutane Vorrichtung kann insbesondere zum Einsatz in eine Insertionsvorrichtung in einer oder mehreren der oben beschriebenen Ausführungsformen eingesetzt werden. Die subkutane Vorrichtung ist dementsprechend eingerichtet, um mit einem Grundkörper einer Insertionshilfe zum Einführen in das Körpergewebe derart zusammenzuwirken, dass zwischen dem Grundkörper und der subkutanen Vorrichtung eine veränderbare Haltekraft erzeugbar ist. Beispielsweise kann es sich bei diesem Grundkörper um den oben beschriebenen, im Wesentlichen starr ausgebildeten Grundkörper der Insertionshilfe handeln. Wie oben dargestellt, kann die subkutane Vorrichtung zu diesem Zweck, beispielsweise in einem implantierbaren Teil, mindestens ein zweites Halteelement umfassen, welches mit dem Grundkörper der Insertionshilfe derart zusammenwirken kann, dass die veränderbare Haltekraft erzeugbar ist. Beispielsweise kann dieses zweite Halteelement mit einem ersten Halteelement der Insertionshilfe zusammenwirken und/oder mit dem gesamten Grundkörper der Insertionshilfe. Wie oben dargestellt, kann das zweite Halteelement beispielsweise auf einem elektrostatischen Prinzip, einem magnetischen Prinzip, insbesondere einem elektromagnetischen Prinzip, und/oder dem Prinzip einer Veränderung einer Ladungsträgerdichte und/oder einer Leitfähigkeit im Bereich mindestens einer Haftoberfläche der subkutanen Vorrichtung basieren. Beispielsweise kann diese Haftoberfläche eine Oberfläche der subkutanen Vorrichtung sein, welche während des Insertierens auf der oben beschriebenen Auflagefläche der Insertionshilfe aufliegt. Auch andere Wirkprinzipien zur Erzeugung einer veränderbaren Haltekraft sind jedoch alternativ oder zusätzlich einsetzbar.

Die erfindungsgemäße Insertionsvorrichtung, die Insertionshilfe und die subkutane Vorrichtung weisen gegenüber bekannten Vorrichtungen der genannten Arten zahlreiche Vorteile auf. Beispielsweise kann auf diese Weise eine steuerbare Insertionsnadel realisiert werden, mittels derer eine subkutane Vorrichtung, beispielsweise ein Sensor, gezielt und zuverlässig implantiert werden kann. Hierbei kann eine physikalische Anbindung von Insertionsnadel und Sensor für das Insertieren beispielsweise eines kontinuierlich messenden Sensors verwendet werden. Wie oben dargestellt, können verschiedene physikalische Mechanismen verwendet werden, beispielsweise elektrostatische Aufladung, die durch Anschließen eines elektrischen Feldes an die Insertionsnadel und den Sensor stattfindet, oder Magnetismus oder ein gezieltes Verändern von Hafteigenschaften einer Haftoberfläche. Letzteres kann, wie oben ausgeführt, beispielsweise durch Anlegen eines elektrischen Feldes erfolgen, welches konduktive organische Polymere so ausrichten kann, dass ihre Hafteigenschaften an Grenzflächen sich ändern und/oder dass eine Ladungsträgerdichte und/oder eine Leitfähigkeit an der Haftoberfläche sich ändert. Die genannten Prinzipien oder andere Prinzipien können als Gemeinsamkeit aufweisen, dass durch Variation von physikalischen Eigenschaften des Verbundes aus Insertionsnadel und Sensor bzw. Insertionshilfe und subkutaner Vorrichtung die Anhaftung dieser beiden Gegenstände gezielt veränderbar ist. Damit unterscheidet sich die Idee der vorliegenden Erfindung vom Stand der Technik, in welchem im Wesentlichen starre oder langsam in ihren eigenen Eigenschaften veränderbare Insertionshilfen verwendet werden. Gegenüber der oben beschriebenen WO 03/080169 unterscheidet sich die Idee der vorliegenden Erfindung darin, dass die Haltekraft gezielt verändert werden kann und beispielsweise während des Einführens auch eine Haltekraft auf die subkutane Vorrichtung ausgeübt werden kann, welche entlang einer Insertionsrichtung, also parallel zu den Längserstreckungsachsen der Insertionshilfe und/oder der subkutanen Vorrichtung, wirken kann. Auf diese Weise kann beispielsweise auch ein Verschieben der subkutanen Vorrichtung auf oder in der Insertionshilfe während des Implantierens oder ein Knicken derselben verhindert werden.

Mittels der beschriebenen Insertionsvorrichtung, der Insertionshilfe und/oder der subkutanen Vorrichtung kann insbesondere ein subkutaner, flexibler Sensor oder eine andere Art subkutaner Vorrichtung minimalinvasiv derart in das Körpergewebe appliziert werden, beispielsweise in die Haut, dass das Stichprofil minimal ausgestaltet ist. Dazu kann die subkutane Vorrichtung und/oder deren implantierbarer Teil während des Einführens (Insertierens) versteift werden. Beim Entfernen der Insertionshilfe kann die subkutane Vorrichtung unverändert in ihrer bestimmungsgemäßen Form verbleiben. Das Zurücklassen der subkutanen Vorrichtung im Gewebe beim Entfernen der Insertionshilfe kann somit zu einem definierten Zeitpunkt, also gesteuert erfolgen. Insbesondere kann durch einen "Auslösemechanismus" ein definierter Zeitpunkt festgelegt werden, zu welchem die subkutane Vorrichtung von der Insertionshilfe gelöst wird, beispielsweise indem gezielt Einfluss auf die veränderbare Haltekraft genommen wird.

Dies kann insbesondere derart ausgestaltet werden, dass die subkutane Vorrichtung, welche, wie oben dargestellt, vorzugsweise ganz oder teilweise flexibel ausgestaltet ist, beispielsweise als flexibler Sensor, während des Insertierens, beispielsweise während des Einstechens, zeitlich definiert durch die Insertionshilfe unterstützt wird. Dies kann gesteuert erfolgen, beispielsweise durch eine Steuerung, welche gezielt Einfluss auf die Haltekraft nimmt. Die Insertionshilfe kann beispielsweise eine der subkutanen Vorrichtung bzw. deren implantierbarem Teil, beispielsweise dem Sensor, in der Kontur ähnliche, flache Struktur aufweisen, welche die subkutane Vorrichtung unterstützt. Die subkutane Vorrichtung, beispielsweise der flexible Sensor, kann dadurch nicht in Folge von Insertionskräften aus ihrer geraden Insertionsbewegung ausweichen. Dabei kann sich die flexible subkutane Vorrichtung an eine steife, dem Sensor von der Kontur her ähnliche, flache Insertionsnadel anschmiegen. Dieses Anschmiegen kann beispielsweise, wie oben dargestellt, durch eine oder mehrere veränderbare Haltekräfte erzeugt werden, beispielsweise durch elektrostatische, elektromagnetische oder steuerbare Adhäsionskräfte bzw. Klebekräfte. Der Begriff der Adhäsion und der Klebung wird dabei im vorliegenden Rahmen im Wesentlichen synonym verwendet. Auch eine Kombination der genannten Haltekräfte und/oder anderer Haltekräfte, welche veränderbar sind, ist möglich. Die gesteuerte Schmiegung zwischen der Insertionsvorrichtung und der subkutanen Vorrichtung kann insbesondere bewirken, dass während des Insertierens die Haltekraft der subkutanen Vorrichtung, beispielsweise des Sensors, an der Insertionshilfe, beispielsweise der Insertionsnadel, die Schiebekräfte übersteigt, welche durch die Haut bzw. das Körpergewebe auf die subkutane Vorrichtung einwirken. Ist die subkutane Vorrichtung, beispielsweise der Sensor, in der Endposition angekommen, so kann die Haltekraft gezielt verändert werden, beispielsweise gezielt abgeschaltet oder sogar umgekehrt (so dass beispielsweise eine Abstoßung erfolgt) werden, so dass beim Entfernen der Insertionshilfe aus dem Körpergewebe die subkutane Vorrichtung in der Endposition verbleibt, also in einer zumindest teilweise implantierten Position. Beispielsweise kann dies dadurch erfolgen, dass eine Reibung zwischen der subkutanen Vorrichtung und der Insertionshilfe reduziert wird und/oder die subkutane Vorrichtung von der Insertionshilfe abgestoßen wird, so dass die Insertionshilfe leicht und ohne die subkutane Vorrichtung aus dem Körpergewebe herausgezogen werden kann.

Die Insertionshilfe, insbesondere die Insertionsnadel, sollte vorzugsweise lediglich die Breite der subkutanen Vorrichtung, insbesondere eines implantierbaren Teils der subkutanen Vorrichtung, beispielsweise eines Sensors, aufweisen und kann dementsprechend relativ dünn ausgestaltet sein. Auf diese Weise kann sichergestellt werden, dass das Einstichprofil nur minimal größer ist als das Sensorprofil alleine und auf jeden Fall geringer als das einer Hohlnadel. Dementsprechend ist es bevorzugt, wenn die Insertionshilfe, insbesondere die Insertionsnadel, senkrecht zur Insertionsrichtung eine maximale Ausdehnung aufweist, welche die maximale Ausdehnung des implantierbaren Teils der subkutanen Vorrichtung nicht oder lediglich geringfügig, beispielsweise um maximal 50%, vorzugsweise um maximal 20% und besonders bevorzugt um maximal 10%, übersteigt. Dies kann beispielsweise dadurch sichergestellt werden, dass die implantierbare Vorrichtung, beispielsweise ein flexibler Sensor, die Auflagefläche vollständig bedeckt, zumindest in der Breite, also senkrecht zur Insertionsrichtung. Grundsätzlich kann die subkutane Vorrichtung jedoch auch ganz oder teilweise gegründet ausgestaltet sein, beispielsweise mit einem abgerundeten, gewinkelten oder gebogenen Profil in einer Schnittebene senkrecht zur Richtung der Insertion. Bevorzugt ist jedoch eine im Wesentlichen ebene Ausgestaltung in dieser Schnittebene.

Auch die oben beschriebene gezielte Beeinflussung von Hafteigenschaften einer Haftoberfläche durch Beeinflussung der Ladungsträgerdichte und/oder der Leitfähigkeit im Bereich dieser Haftoberfläche eröffnet neue Anwendungsmöglichkeiten, insbesondere bei Verwendung organischer halbleitender und/oder leitender Materialien, wie beispielsweise konjugierter Polymere oder niedermolekularer organischer Substanzen mit konjugierten $\pi$-Elektronensystemen. So können beispielsweise polymerelektronische Materialien kostengünstig hergestellt und eingesetzt werden, auch in großtechnischen Massenprozessen. Auf diese Weise lassen sich beispielsweise subkutane Vorrichtungen, insbesondere implantierbare Sensoren, kostengünstig und flexibel herstellen. Polymerelektronische Materialien können beispielsweise gezielt auf neue, spezifische Eigenschaften hin optimiert und entwickelt werden. So ist es beispielsweise vorstellbar, dass ein Polymer durch Anlegen einer elektrischen Spannung seine Ausrichtung, und damit seine lokale Ladungsträgerdichte, und/oder seine Ladungsträgerdichte oder Leitfähigkeit, gezielt verändert. Damit lassen sich Hafteigenschaften durch gezieltes Steuern von Adhäsionskräften einstellen.

Kurze Beschreibung der Zeichnungen

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:

Figuren 1A und 1B    verschiedene Darstellungen eines ersten Ausführungsbeispiels einer Insertionsvorrichtung mit einer elektrostatischen Haltekraft;

Figuren 2A und 2B    verschiedene Ansichten eines zweiten Ausführungsbeispiels einer Insertionsvorrichtung mit einer magnetischen Haltekraft;

Figuren 3A bis 3C    verschiedene Profile einer Insertionsvorrichtung;

Figur 4    eine Insertionsvorrichtung mit einer erhöhten Einführspitze; und

Figur 5                    ein Ausführungsbeispiel einer Insertionsvorrichtung mit einer durch organische elektronische Strukturen erzeugten, veränderlichen Haltekraft.

Ausführungsbeispiele

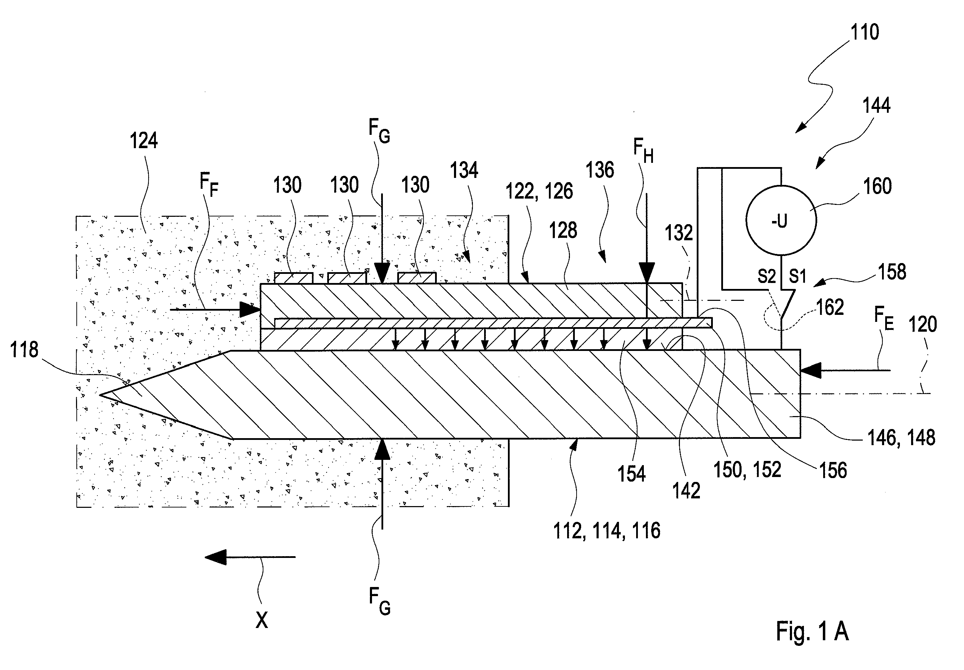

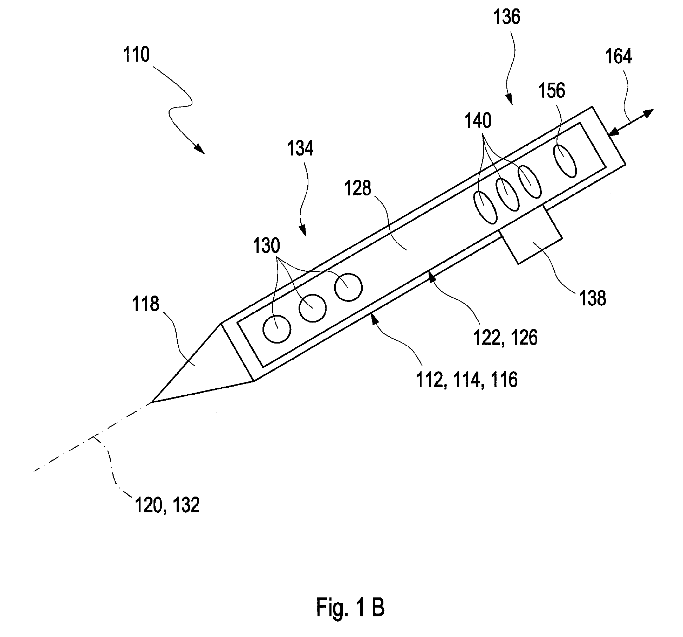

In den Figuren 1A und 1B ist ein erstes Ausführungsbeispiel einer erfindungsgemäßen Insertionsvorrichtung 110 in verschiedenen Darstellungen gezeigt. Dabei zeigt Figur 1A eine Schnittdarstellung in einer Schnittebene parallel zu einem mit "x" bezeichneten Einstichweg, und Figur 1B zeigt eine Draufsicht auf die Insertionsvorrichtung 110.

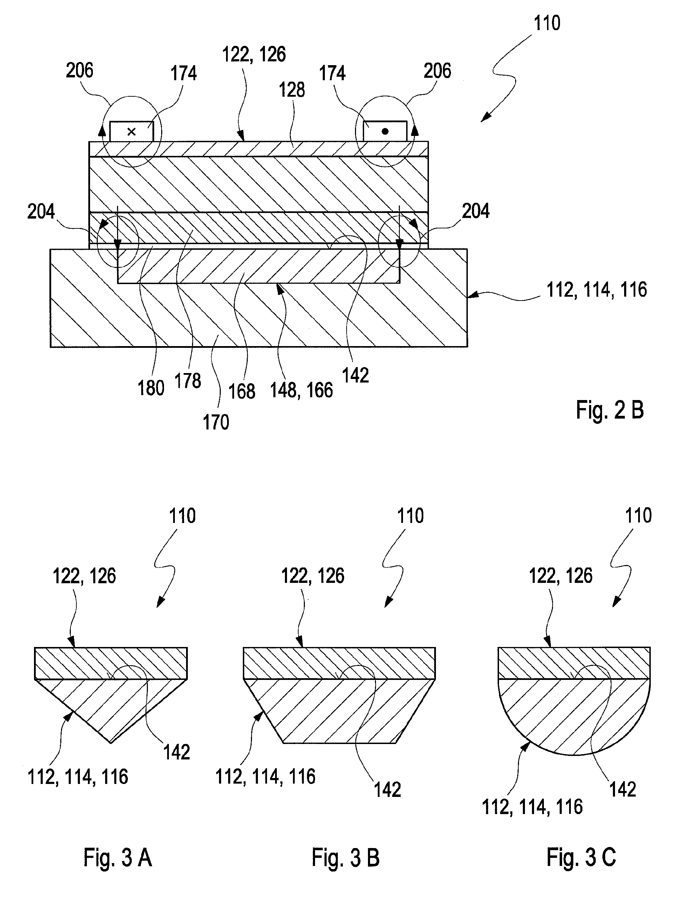

Die Insertionsvorrichtung 110 umfasst eine Insertionshilfe 112 mit einem im Wesentlichen starr ausgebildeten Grundkörper 114. Der Grundkörper 114 ist hier als Insertionsnadel 116 ausgebildet, beispielsweise in Form einer Vollnadel, beispielsweise einer aus Stahl gefertigten Vollnadel. Die Insertionsnadel 116 weist eine Einführspitze 118 auf, welche hier exemplarisch als Rundspitze dargestellt ist. Auch andere Ausgestaltungen sind jedoch grundsätzlich möglich, beispielsweise die unten in den Figuren 3A bis 4 beschriebenen Ausgestaltungen. Die Insertionsnadel 116 ist als langgestreckter, starrer Grundkörper 114 ausgestaltet, welcher sich im Wesentlichen in Richtung des Einstichwegs x erstreckt, mit einer Längserstreckungsachse 120.

Weiterhin weist die Insertionsvorrichtung 110 eine subkutane Vorrichtung 122 auf, welche mittels der Insertionsvorrichtung 110 in ein Körpergewebe 124 insertiert werden soll. Das Körpergewebe 124 ist in Figur 1A lediglich symbolisch dargestellt. Die subkutane Vorrichtung 122 ist in dem dargestellten Ausführungsbeispiel exemplarisch als subkutaner Sensor 126 ausgestaltet, welcher zur Erfassung mindestens eines Analyten in dem Körpergewebe 124 bzw. einer Körperflüssigkeit in dem Körpergewebe 124 dient. Wie oben dargestellt, sind jedoch auch andere Ausgestaltungen möglich. Der subkutane Sensor 126 weist einen Träger 128 auf, auf welchem in dem dargestellten Ausführungsbeispiel exemplarisch drei Sensorelektroden 130 angeordnet sind, beispielsweise derart, dass diese Sensorelektroden 130 dem Körpergewebe 124 zuweisen. Die Sensorelektroden 130 sind dabei beispielsweise kreisförmig ausgestaltet, wobei auch andere Geometrien möglich sind, beispielsweise eine ovale oder polygonale Geometrie. Auch eine andere Anzahl von Sensorelektroden ist möglich. Beispielsweise können die Sensorelektroden 130 mindestens eine Arbeitselektrode, mindestens eine Gegenelektrode und mindestens eine Referenzelektrode umfassen, um elektrochemisch mindestens einen Analyten in dem Körpergewebe 124 und/oder einer Körperflüssigkeit nachzuweisen. Die mindestens eine Gegenelektrode und die mindestens eine Referenzelektrode können auch ganz oder teilweise zu einer gemeinsamen Elektrode zusammengefasst seien. Die Sensorelektroden 130 können neben beispielsweise metallischen Elektroden weiterhin beispielsweise ein oder mehrere Redoxsysteme und/oder Enzyme umfassen. Derartige Sensorelektroden 130 sind aus dem Bereich der elektrochemischen Analyterfassung grundsätzlich aus dem Stand der Technik bekannt.

Der Träger 128 ist beispielsweise flexibel ausgestaltet und kann beispielsweise einen Mehrschichtaufbau umfassen. Der Träger 128 kann beispielsweise ganz oder teilweise aus einem Kunststoffmaterial und/oder einem Papiermaterial und/oder einem keramischen Material und/oder einem Laminatmaterial hergestellt sein und kann sich ebenfalls entlang einer Längserstreckungsachse 132 erstrecken, welche beispielsweise während des Insertierens, wie in Figur 1A dargestellt, parallel zur Längserstreckungsachse 120 angeordnet sein kann. Die subkutane Vorrichtung 122 wird dabei teilweise in das Körpergewebe 124 insertiert, mit einem implantierbaren Teil 134 und einem nicht-implantierten Teil 136. Im Bereich des nicht-implantierten Teils 136 kann die subkutane Vorrichtung 122 beispielsweise einen Halter 138 aufweisen, welcher insbesondere in Figur 1B erkennbar ist. Mittels dieses Halters 138 kann die subkutane Vorrichtung 122 beispielsweise gehalten werden und/oder nach Verwendung aus dem Körpergewebe 124 herausgezogen werden. Im Bereich des nicht-implantierten Teils 136 kann die subkutane Vorrichtung 122 beispielsweise Sensorkontakte 140 umfassen, beispielsweise jeweils ein Sensorkontakt 140 je Sensorelektrode 130. Diese Sensorkontakte 140 können beispielsweise über in den Figuren 1A und 1B nicht dargestellte Elektrodenzuleitungen, beispielsweise innerhalb eines Schichtaufbaus des Trägers 128, mit den Sensorelektroden 130 verbunden sein, so dass die Sensorelektroden 130 elektrisch kontaktiert bzw. deren Potenziale abgegriffen werden können.

Der im Wesentlichen streifenförmig ausgestaltete, flexible subkutane Sensor 126 liegt in dem in den Figuren 1A und 1B dargestellten Ausführungsbeispiel flach auf einer Auflagefläche 142 der Insertionshilfe 112 auf. Auch andere Ausgestaltungen sind jedoch grundsätzlich möglich.

Ein Gedanke der vorliegenden Insertionsvorrichtung 110 gemäß dem dargestellten Ausführungsbeispiel besteht darin, eine veränderbare Haltekraft zwischen der Insertionshilfe 112 und der subkutanen Vorrichtung 122 zu erzeugen. Zu diesem Zweck weist die Insertionsvorrichtung 110 in dem dargestellten Ausführungsbeispiel eine Steuerung 144 auf, welche beispielsweise Bestandteil der Insertionshilfe 112 sein kann und/oder extern angeordnet sein kann, beispielsweise in einem wiederverwertbaren Teil der Insertionsvorrichtung 110. Die Insertionsnadel 116 ist in diesem Ausführungsbeispiel beispielsweise metallisch ausgestaltet und wirkt insgesamt als erste Elektrode 146 und damit als erstes Halteelement 148. Die subkutane Vorrichtung 122 weist ihrerseits eine zweite Elektrode 150 als zweites Halteelement 152 auf. Beispielsweise kann diese zweite Elektrode 150 in Form einer metallischen Kaschierung und/oder einer flexiblen Kupferbeschichtung auf den Träger 128 aufgebracht sein und/oder in den Träger 128 integriert sein. Verschiedene Ausgestaltungen sind möglich. So kann beispielsweise die Insertionsnadel 116 auch ganz oder teilweise, anstelle der Verwendung von Metallen, mit organischen Polymeren, welche leitende Eigenschaften aufweisen, beschichtet sein und so eine erste Elektrode 146 bilden. Auch bei der subkutanen Vorrichtung 122 ist die Verwendung organischer leitender Materialien als Materialien der zweiten Elektrode 150 denkbar.

Die Materialien der Insertionshilfe 112 und/oder der subkutanen Vorrichtung 122 sind vorzugsweise ganz oder teilweise sterilisierbar ausgestaltet, beispielsweise mittels einer Strahlung, insbesondere einer oder mehrerer der folgenden Strahlungen: UV-Strahlung, Röntgenstrahlung, Gammastrahlung, Elektronenstrahlung.

Weiterhin weist die Insertionsvorrichtung 110 mindestens einen zwischen der ersten Elektrode 146 und der zweiten Elektrode 150 angeordneten Isolator 154 auf. Beispielsweise kann dieser Isolator 154 ganz oder teilweise Bestandteil der Insertionshilfe 112 und/oder ganz oder teilweise Bestandteil der subkutanen Vorrichtung 122 sein. Insbesondere können die Insertionsnadel 116 und/oder die subkutane Vorrichtung 122 zur Vermeidung von Kurzschlüssen im Gewebe ringsherum mit einem Isolator 154 versehen sein, beispielsweise einer entsprechenden Beschichtung.

Die erste Elektrode 146 und die zweite Elektrode 150 können während des Insertionsvorgangs mit der Steuerung 144 verbunden sein. Zu diesem Zweck kann die subkutane Vorrichtung 122, wie beispielsweise in Figur 1B erkennbar, beispielsweise einen Feldplattenkontakt 156 umfassen, über welchen eine entsprechende Verbindung zwischen der Steuerung 144 und der beispielsweise als Feldplatte ausgestalteten zweiten Elektrode 150 hergestellt werden kann. Entsprechend kann auch die Insertionshilfe 112, insbesondere die Insertionsnadel 116, mit der Steuerung 144 verbunden werden. Diese Verbindung kann permanent sein, kann jedoch auch nach erfolgter Insertion ganz oder teilweise getrennt werden.

Die Steuerung 144 umfasst, wie in Figur 1A dargestellt, vorzugsweise eine Umschaltvorrichtung 158, mittels derer eine Potenzialdifferenz zwischen der ersten Elektrode 146 und der zweiten Elektrode 150 veränderbar ist, so dass eine elektrostatische Haltekraft zwischen der subkutanen Vorrichtung 122 und der Insertionshilfe 112 veränderbar ist. Die Umschaltvorrichtung 158 ist in dem dargestellten Ausführungsbeispiel eingerichtet, um wahlweise die erste Elektrode 146 und die zweite Elektrode 150 über eine Spannungsquelle 160 miteinander zu verbinden oder über eine Kurzschlussverbindung 162. Auch andere Ausgestaltungen sind möglich, mittels derer ein Potenzialunterschied zwischen der ersten Elektrode 146 und der zweiten Elektrode 150 veränderbar ist. In dem dargestellten Ausführungsbeispiel gemäß Figur 1A bewirkt die dargestellte Schaltstellung, welche hier mit $S_1$ bezeichnet ist, eine anziehende Haltekraft zwischen der subkutanen Vorrichtung 122 und der Insertionshilfe 112, wohingegen die gestrichelt dargestellte Schaltstellung $S_2$ der Umschaltvorrichtung 158 eine Haltekraft 0 bzw. sogar eine Abstoßung zwischen der Insertionsnadel 116 und der subkutanen Vorrichtung 122 bewirken würde. Die Spannungsquelle 160 kann insbesondere eine Gleichspannungsquelle umfassen. In der Schaltstellung $S_1$ wirkt somit eine anziehende elektrostatische Kraft. Die Spannung bleibt vorzugsweise während dem Einstechvorgang angelegt. Bevor die Insertionsnadel 116 aus dem Körpergewebe 124 herausgezogen wird, wird die Spannungsquelle 160 abgeschaltet bzw. die Umschaltvorrichtung 158 in die Schaltstellung $S_2$ gebracht. Dadurch werden die Elektroden 146, 150 beispielsweise kurzgeschlossen. In diesem Fall wirkt keine Haltekraft mehr oder die Elektroden 146, 150 stoßen sich aufgrund gleicher Potenziale ab. Die Haftreibung wird so reduziert, und die Insertionsnadel 116 kann, gegen den Andruck des Körpergewebes 124, leicht herausgezogen werden. Dies kann beispielsweise mittels einer entsprechenden Einstechvorrichtung und/oder Ausziehvorrichtung erfolgen, welche optional auch einen elektrischen Kontakt bereitstellen kann, und welche symbolisch in Figur 1B mit der Bezugsziffer 164 bezeichnet ist.

Im Folgenden sollen die Kraftverhältnisse der auf die Insertionsvorrichtung 110 wirkenden Kräfte dargestellt werden. Diese Kräfte sind teilweise in Figur 1A dargestellt. Dabei bezeichnet $F_G$ die Kraft, welche durch das Gewebe auf die Insertionsvorrichtung 110 ausgeübt wird, $F_H$ bezeichnet die durch das elektrische Feld hervorgerufene Haltekraft, $F_E$ bezeichnet die Einstechkraft während des Insertionsvorgangs, und $F_F$ bezeichnet die frontale, auf die subkutane Vorrichtung 122 während des Insertionsvorgangs wirkende Kraft.

Für die elektrostatische Kraft zwischen zwei idealen, plattenförmigen Elektroden 146, 150 gilt folgende Relation:

$$F_H = \frac{1}{2} * \frac{\varepsilon_0\, \varepsilon_r * A * U^2}{a^2} \quad (1)$$

Dabei bezeichnet $\varepsilon_0$ die Dielektrizitätskonstante, welche $8{,}85 * 10^{-12}$ As/Vm beträgt, $\varepsilon_r$ bezeichnet die relative Dielektrizitätskonstante, welche typischerweise zwischen 4 und 80 liegt, und welche dimensionslos ist, A bezeichnet die wirksame Elektrodenfläche der Elektroden 146 und 150, welche beispielsweise zwischen 5 und 50 mm² liegen kann, insbesondere bei 20 mm², a bezeichnet den Abstand zwischen den Elektroden 146, 150, welcher beispielsweise zwischen 10 μm und 100 μm liegen kann, insbesondere bei 50 μm, und U bezeichnet die Spannung zwischen den Elektroden 146, 150, welche beispielsweise zwischen 1 und 100 Volt liegen kann, insbesondere bei 10 Volt. Bei den angegebenen Werten mit einer Fläche A von 20 mm², einem Abstand a von 50 μm und einer Spannung von 10 Volt beträgt die Haltekraft ungefähr 0,3 mN, ist also vergleichsweise gering. Um diese Haltekraft $F_H$ zu erhöhen, ist die Erhöhung der Spannung U und/oder eine Reduzierung des Abstandes a geeignet.

Für das Einstechen der Insertionsvorrichtung 110, insbesondere der Insertionsnadel 116 und des subkutanen Sensors 126, muss folgende Einstechkraft $F_E$ aufgewendet werden:

$$F_E = \mu_{1,2} * F_G + \mu_{3,4} * F_G + F_F \qquad (2).$$

Diese Relation (2) berücksichtigt vereinfachend lediglich die Gleitreibung und einen statischen Zustand. Dabei bezeichnen $\mu_{1,2}$ und $\mu_{3,4}$ die Gleitreibungskoeffizienten zwischen der subkutanen Vorrichtung 122 und dem Gewebe 124 ($\mu_{1,2}$) bzw. zwischen der Insertionshilfe 112 und dem Gewebe 124 ($\mu_{3,4}$).

Damit die subkutane Vorrichtung 122 während des Insertionsvorgangs auf der Insertionshilfe 112 haftet, muss gelten:

$$(\mu_{1,2} * F_G + F_F) << \mu_{2,3} * F_H. \qquad (3)$$

Dabei bezeichnet $\mu_{2,3}$ den Reibungskoeffizienten zwischen der subkutanen Vorrichtung 122 und der Insertionshilfe 112, welcher, multipliziert mit der Haltekraft $F_H$, die Abreibungskraft der subkutanen Vorrichtung 122 auf der Insertionshilfe 112 charakterisiert, die einem Verrutschen der subkutanen Vorrichtung 122 auf der Insertionshilfe 112 während des Einstichvorgangs entgegenwirkt. Ist die oben genannte Relation (3) erfüllt, und ist die Insertionsnadel 116 genügend steif, so bleibt die Insertionsvorrichtung 110 während des Insertionsvorgangs gestreckt. Typische Haftreibungskoeffizienten $\mu_{1,2}$ und/oder $\mu_{3,4}$ liegen in der Größenordnung von 0,24, beispielsweise für Messing auf Holzgewebe, mit einem dazwischen liegendem Wasserfilm.

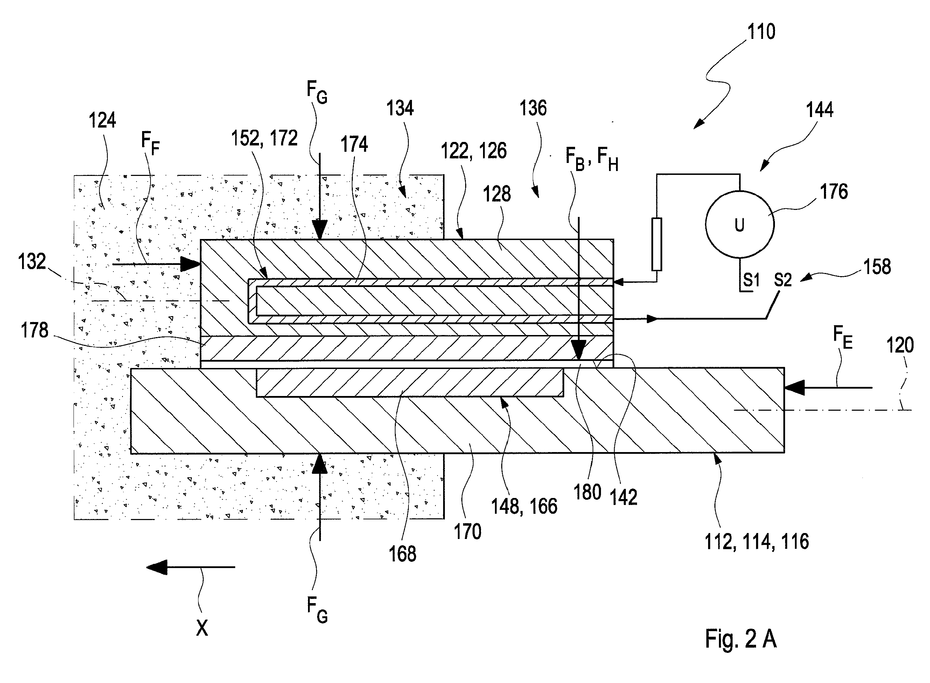

Während das Ausführungsbeispiel in den Figuren 1A und 1B eine Erzeugung einer veränderbaren Haltekraft aufgrund elektrostatischer Prinzipien zeigt, ist in den Figuren 2A und 2B ein Ausführungsbeispiel einer Insertionsvorrichtung 110 dargestellt, welches auf einer magnetischen Erzeugung einer Haltekraft basiert. Die Erzeugung ist in den Figuren 2A und 2B lediglich schematisch dargestellt, wobei Figur 2A wiederum eine Schnittdarstellung durch die Insertionsvorrichtung 110 parallel zu einem Einstichweg x zeigt (analog zur Darstellung in Figur 1A) und wobei Figur 2B eine Schnittdarstellung senkrecht zur Bildebene in Figur 2A zeigt.

Die Insertionsvorrichtung 110 umfasst wiederum eine Insertionshilfe 112, welche wiederum in einen Grundkörper 114 in Form einer Insertionsnadel 116 aufweisen kann. Diesbezüglich kann weitgehend auf die obige Beschreibung der Figuren 1A und 1B verwiesen werden. Eine Einführspitze 118 ist in diesem Ausführungsbeispiel nicht gezeigt, kann jedoch gleichwohl vorhanden sein.

Weiterhin umfasst die Insertionsvorrichtung 110 wiederum eine subkutane Vorrichtung 122, welche beispielsweise wiederum als subkutaner Sensor 126 ausgestaltet sein kann, und welche beispielsweise in ein Körpergewebe 124 implantiert werden soll. Für die mögliche Ausgestaltung des subkutanen Sensors 126 kann beispielsweise auf die obige Beschreibung der Figuren 1A und 1B verwiesen werden.

Zur Erzeugung einer veränderbaren Haltekraft zwischen der subkutanen Vorrichtung 122 und der Insertionshilfe 112 weist in diesem Ausführungsbeispiel die Insertionshilfe 112 wiederum ein erstes Halteelement 148 auf, und die subkutane Vorrichtung 122 entsprechend ein zweites Halteelement 152. Wie oben dargestellt, sollen die Halteelemente 148, 152 eine Haltekraft $F_H$ erzeugen, welche in diesem Fall vollständig oder teilweise als magnetische Haltekraft $F_B$ ausgestaltet ist. Dementsprechend sind die Halteelemente 148, 152 als magnetische Halteelemente ausgestaltet. Dies kann auf verschiedenen Weisen erfolgen, durch Verwendung von Permanentmagneten und/oder durch Verwendung von Elektromagneten, welche auch in beliebiger Weise kombiniert werden können. In den Figuren 2A und 2B ist dies in einer Weise ausgestaltet, in welcher das erste Halteelement 148 einen Permanentmagneten 166 mit einem magnetischen Pol 168 und einem magnetischen Gegenpol 170 umfasst. Beispielsweise kann die Insertionshilfe 112 vollständig aus einem magnetisierbaren bzw. magnetischen Material hergestellt sein, welches entsprechend magnetisiert ist. Alternativ oder zusätzlich kann das erste Halteelement 148 auch einen in ein Trägermaterial der Insertionshilfe 112 eingelassenen Permanentmagneten oder mehrere derartiger Permanentmagnete und/oder mindestens ein magnetisch leitendes Ankerblech umfassen.

Das zweite Halteelement 152 umfasst in dem dargestellten Ausführungsbeispiel ein elektromagnetisches Halteelement 172, welches in diesem Ausführungsbeispiel eine einfache Leiterbahnschleife 174 umfasst. Auch komplexere Anordnungen sind selbstverständlich möglich. Diese Leiterbahnschleife 174 ist in Figur 2A lediglich symbolisch dargestellt und kann beispielsweise, wie in Figur 2B zu erkennen ist, auf einen Träger 128 der subkutanen Vorrichtung 122 aufgebracht und/oder in diesen eingebracht sein. Der Träger 128 kann beispielsweise ein isolierendes Material umfassen, beispielsweise einen Kunststoff, ein Papier, eine Keramik oder auch ein Laminat. Grundsätzlich ist auch die Verwendung leitender Materialien möglich. In der Schnittdarstellung in Figur 2B ist eine mögliche Stromrichtung durch die Leiterbahnschleife 174 dargestellt, wobei der Strom im linken Leiterbahnschleifenabschnitt in die Zeichenebene hineinfließt und im rechten Leiterbahnabschnitt aus der Zeichenebene heraus. Auch eine andere Ausgestaltung ist jedoch möglich. Die Leiterbahnschleife kann durch eine Steuerung 144 mit Strom beaufschlagt werden, wobei die Steuerung 144 beispielsweise wiederum eine Spannungsquelle und/oder eine Stromquelle 176 umfassen kann, sowie gegebenenfalls ein oder mehrere weitere Bauelemente, beispielsweise Widerstände, Kondensatoren, Dioden oder auch aktive elektronische Bauelemente. Verschiedene Ausgestaltungen sind möglich. Die Ausgestaltung einer Steuerung 144 zur Steuerung eines elektromagnetischen Halteelements 172 ist dem Fachmann grundsätzlich bekannt.

Die Steuerung 144 kann eingerichtet sein, um den Strom durch die Leiterbahnschleife 174 gezielt einzustellen bzw. zu beeinflussen. In dem in Figur 2A gezeigten Ausführungsbeispiel der Steuerung 144 erfolgt dies durch ein reines digitales Ein- bzw. Ausschalten. Dabei kann wiederum eine Umschaltvorrichtung 158 vorgesehen sein, um von einem Schaltzustand $S_1$, in welchem die Leiterbahnschleife 174 mit einem Strom beaufschlagt ist, in einen Schaltzustand $S_2$ zu schalten, in welchem die Umschaltvorrichtung 158 geöffnet ist und kein Strom fließt. Auch eine andere Ausgestaltung ist grundsätzlich möglich. So kann durch die Steuerung 144 allgemein beispielsweise der Betrag der Stromstärke und/oder die Richtung des Stroms durch die Leiterbahnschleife 174 beeinflusst werden, so dass, in Zusammenwirkung mit dem ersten Halteelement 148, eine anziehende oder abstoßende Kraft oder die Kraft 0 zwischen den beiden Halteelementen 148 und 152 generiert werden kann. Anschaulich kann dies beispielsweise dadurch erläutert werden, dass der Permanentmagnet 166 magnetische Feldlinien erzeugt, welcher ihn Figur 2B symbolisch mit der Bezugsziffer 204 bezeichnet sind. Diese werden durch das Substrat-Ankerblech 178 verkürzt, wodurch eine Haltekraft entsteht. Der Strom durch die Leiterbahnschleife 174 kann derart eingestellt werden, dass die Leiterbahnschleife 174 umgebende magnetische Feldlinien 206 dem Feld des Permanentmagneten 166 entgegenwirken oder gleichgerichtet sind, so dass eine resultierende magnetische Gesamtkraft eingestellt werden kann und beispielsweise eine abstoßende Wirkung erreicht werden kann. So kann beim Einstechen bzw. Einführen der Insertionsvorrichtung 110 in das Körpergewebe 124 eine anziehende Haltekraft eingestellt werden, wohingegen nach dem Einstechen, zum Lösen der subkutanen Vorrichtung 122 von der Insertionshilfe 112, die Kraft 0 oder sogar eine abstoßende Kraft zwischen der subkutanen Vorrichtung 122 und der Insertionshilfe 112 eingestellt werden kann.

Unterhalb des Trägers 128 ist in dem Schichtaufbau der in der subkutanen Vorrichtung 122 in dem dargestellten Ausführungsbeispiel in den Figuren 2A und 2B exemplarisch ein Substrat-Ankerblech 178 angeordnet. Unter diesem Substrat-Ankerblech 178, welches beispielsweise aus einem magnetischen Material, beispielsweise einem weichmagnetischen Material, hergestellt sein kann oder ein derartiges Material umfassen kann, kann beispielsweise ein Luftspalt 180 sein, der optional auch ganz oder teilweise durch mindestens einen Isolator ausgefüllt sein kann. Auf diese Weise kann eine Magnetkraft $F_B$ beeinflusst werden. Der Luftspalt 180 ist dabei lediglich in der Darstellung gemäß Figur 2A gezeigt, kann jedoch grundsätzlich auch in der Darstellung gemäß Figur 2B eingefügt werden.

Ohne Strom durch die Leiterbahnschleife 174 wirkt beispielsweise eine magnetische Kraft des Permanentmagneten 166 des ersten Halteelements 148 auf das Substrat-Ankerblech 178 des zweiten Halteelements 152. Die Stromrichtung durch die Leiterbahnschleife 174 kann beispielsweise derart gewählt werden, dass diese magnetische Haltekraft durch das Magnetfeld, welches die Leiterbahnschleife 174 erzeugt, geschwächt wird, indem dieses dem Magnetfeld des Permanentmagneten 166 entgegenwirkt. Dadurch wird die Haltekraft kompensiert, aufgehoben oder sogar überkompensiert. Auf diese Weise kann durch Einstellen der Stromstärke durch die Leiterbahnschleife 174 mittels der Steuerung 144 die magnetische Haltekraft beeinflusst werden.

Es wird darauf hingewiesen, dass die dargestellte Ausführungsform lediglich eine von vielen Möglichkeiten ist, wie magnetische Halteelemente eingesetzt werden können. So können das erste Halteelement 148 und/oder das zweite Halteelement 152 allgemein eines oder mehrere der folgenden magnetischen Halteelemente umfassen: Ein elektromagnetisches Halteelement, beispielsweise eine Leiterbahnschleife; einen Permanentmagneten; ein Ankerblech aus einem magnetischen Material, beispielsweise einem weichmagnetischen Metall. Das erste und das zweite Halteelement können dann beispielsweise jeweils selbst magnetische Felder erzeugen und/oder lediglich eines dieser Halteelemente 148, 152 kann ein magnetisches Feld erzeugen, welches das jeweils andere der Halteelemente 148, 152 anziehen oder abstoßen kann. Verschiedene Kombinationsmöglichkeiten sind denkbar.

Im dargestellten Ausführungsbeispiel kann die Insertionsnadel 116 beispielsweise, wie oben erläutert, ganz oder teilweise als Dauermagnet oder Permanentmagnet 166 ausgestaltet sein, beispielsweise indem der magnetische Pol 168 als Nordpol ausgestaltet wird und der magnetische Gegenpol 170 als Südpol oder umgekehrt. Der subkutane Sensor 126 bzw. die subkutane Vorrichtung 122 können zumindest teilweise aus einem leitenden Material hergestellt sein. Grundsätzlich wäre auch beispielsweise die Verwendung von Edelstahl möglich, ist jedoch weniger geeignet, da Edelstahl lediglich schwach magnetisch ist. Auf diese Weise kann das Substrat-Ankerblech 178 gebildet werden, welches im Bereich der magnetischen Pole 168, 170 den Anker bildet. Hierdurch werden die magnetischen Feldlinien verkürzt und bewirken dadurch die Haltekraft während des Einstechens. Ist der subkutane Sensor 126 bzw. die subkutane Vorrichtung 122 vor Ort, so wird auf die Leiterbahnschleife 174, die auf dem subkutanen Sensor 126 hin- und zurückläuft, beispielsweise ein Stromimpuls aufgeprägt. Dieser Stromimpuls kann nun eine Kraft bewirken, die dem statischen Magnetfeld in der Nadel entgegenwirkt und die Haltekraft reduziert oder sogar aufhebt. Die Insertionsnadel 116 kann dann aus dem Gewebe herausgezogen werden, während der subkutane Sensor 126 in dem Körpergewebe 124 verbleibt. Der Stromimpuls kann beispielsweise hinsichtlich seines zeitlichen Verlaufs und/oder seiner Form mit der Ausziehbewegung der Insertionsnadel 116 synchronisiert werden, beispielsweise durch eine entsprechende elektronische Schaltung, welche die Ausziehbewegung detektiert und/oder bewirkt. Auf diese Weise kann beispielsweise ein zeitlich optimales Zusammenspiel der Kräfte realisiert werden, und die eingesetzte Energie kann effizient eingesetzt werden. Dies ist insbesondere bei einem Batteriebetrieb, beispielsweise einer Insertionsvorrichtung 110 und/oder einer Insertionshilfe 112, beispielsweise einem Reusable, mit Batteriebetrieb besonders bevorzugt. Durch die Möglichkeit einer aktiven Steuerung kann somit ein optimales zeitliches Zusammenspiel der Kräfte beim Lösen und Herausziehen erreicht werden, so dass beispielsweise auch dynamische Lösevorgänge und/oder Losbrechvorgänge wirkungsvoller realisiert werden können. Auf diese Weise können auch mit sehr kurzen Strompulsen bereits gute Ergebnisse bewirken, was insbesondere bei einem Reusable, bei welchem die zur Verfügung stehende Energie in der Regel begrenzt ist, von Vorteil ist.

Wiederum lassen sich, in analoger Weise zu dem Ausführungsbeispiel gemäß den Figuren 1A und 1B, die Kraftverhältnisse zumindest näherungsweise berechnen. So wirkt zwischen dem ersten Halteelement 148 und dem zweiten Halteelement 152 bei ausschließlicher Wirkung des Permanentmagneten 166 folgende Kraft:

$$F_{B,P} = \frac{B^2 * A}{2\mu}. \qquad (4)$$

Dabei bezeichnet B die magnetische Induktion des Permanentmagneten 166, A die Fläche des Luftspalts 180 und $\mu$ die magnetische Permeabilität, welche sich nach $\mu = \mu_0 * \mu_r$ aus der Permeabilität des Vakuums $\mu_0$ und der relativen Permeabilität $\mu_r$ des Materials im Luftspalt 180 zusammensetzt.

Dieser Kraft des Permanentmagneten $F_{B,P}$ wirkt die magnetische Kraft der Leiterbahnschleife 174 entgegen, welche im Folgenden als $F_{B,L}$ bezeichnet wird:

$$F_{B,L} = \frac{\mu * l * I^2}{4 * \pi * h} \qquad (5)$$

Dabei bezeichnet l die Länge der Leiterbahnschleife 174, I den Strom durch die Leiterbahnschleife 174, $\mu$ wiederum die magnetische Permeabilität sowie h den Abstand der Leiterbahnschleife 174 zum magnetischen Stoff, auf welchen die Leiterbahnschleife 174 magnetisch einwirkt, beispielsweise zum Permanentmagneten 166. Die Kräfte $F_{B,P}$ und $F_{B,L}$ nach den Gleichungen (4) und (5) oben addieren sich zur gesamten magnetischen Haltekraft

$$F_B = F_H = F_{B,P} + F_{B,L}. \quad (6)$$

Entsprechend kann durch Wahl der Richtung (d.h. des Vorzeichens) und der Stärke des Stroms I sowie durch geeignete Auswahl der Materialien die jeweils wirkende Haltekraft aktuell durch die Steuerung 144 beeinflusst werden, von einer anziehenden Haltekraft $F_H$ über die Haltekraft $F_H = 0$ bis hin zu einer abstoßenden Haltekraft $F_H$.

Entsprechend kann wiederum während des Einstechens die Bedingung gelten, dass die Haltekraft $F_H$ bzw. die durch diese Haltekraft bedingte Haftreibungskraft, die beim Insertieren auf die subkutane Vorrichtung 122 einwirkende Gesamtkraft erheblich übersteigen muss, damit die subkutane Vorrichtung 122 beim Insertieren nicht verbogen oder von der Insertionshilfe 112 abgelöst wird. Die oben dargestellte Formel (3) ist entsprechend anzuwenden, wobei in diesem Fall $F_H$ durch die in Formel (6) beschriebene gesamte magnetische Haltekraft zu ersetzen ist.

In den oben beschriebenen Ausführungsbeispielen in den Figuren 1A bis 2B wurde angenommen, dass die subkutane Vorrichtung 122 zumindest teilweise, insbesondere mit ihrem implantierbaren Teil 134, auf einer Auflagefläche 142 der Insertionshilfe 112 aufliegt. Zu diesem Zweck kann die Auflagefläche 142 beispielsweise abgeflacht ausgestaltet sein, beispielsweise als Abflachung an einer Seite der Insertionsnadel 116, welche sich beispielsweise über die gesamte Länge der Insertionsnadel 116 oder lediglich über einen Teil der Länge erstrecken kann. Vorzugsweise ist diese Auflagefläche 142 eben ausgestaltet. Von der Dimensionierung her ist vorzugsweise die subkutane Vorrichtung 122 in ihrer Breite auf die Breite der Auflagefläche 142 angepasst, so dass die maximale Breite des Einstichs in das Körpergewebe 124 die maximale Breite der subkutanen Vorrichtung 122 bzw. des implantierbaren Teils 134 der subkutanen Vorrichtung 122 nicht oder nur unwesentlich übersteigt.

Wie in den Figuren 3A bis 3C dargestellt ist, kann die Insertionshilfe 112, insbesondere deren Grundkörper 114, insbesondere im Bereich der Auflagefläche 142, derart profiliert ausgestaltet sein, dass die Stabilität der Insertionshilfe 112 beim Insertieren erhöht wird. Beispielsweise ist in Figur 3A ein dreieckförmiges Profil dargestellt, in Figur 3B ein trapezförmiges Profil und in Figur 3C ein kreissegmentförmiges Profil, beispielsweise ein halbkreisförmiges Profil. Hierdurch kann beispielsweise eine Durchbiegung der Insertionsnadel 116 während des Insertierens verhindert werden.

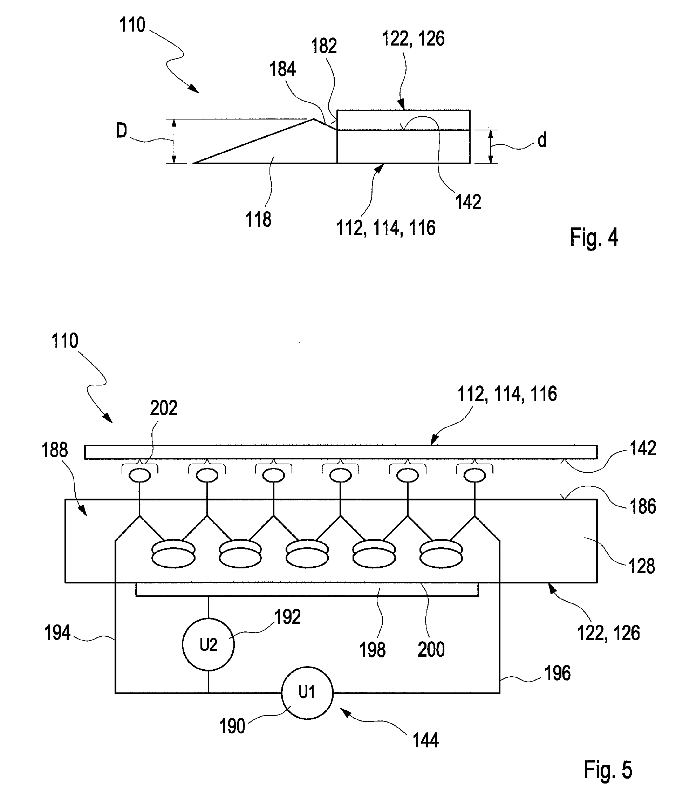

Wie oben dargestellt, kann die Insertionshilfe 112, insbesondere deren Grundkörper 114 bzw. die Insertionsnadel 116 optional eine Einführspitze 118 aufweisen. Dies ist in Figur 4 symbolisch dargestellt. Dabei ist auch dargestellt, dass die maximale Breite der Einführspitze 118, welche in Figur 4 mit D bezeichnet ist, die maximale Breite des Grundkörpers 114 im Bereich der Auflagefläche 142, welche mit d bezeichnet ist, übersteigt. Damit überdeckt die Einführspitze 118 zumindest teilweise eine Stirnfläche 182 der subkutanen Vorrichtung 122. Durch diesen speziell geformten "Bug" lässt sich die während des Insertierens auf die subkutane Vorrichtung 122 einwirkende frontale Kraft $F_F$ (siehe beispielsweise Figuren 1A und 2A) reduzieren. Gleichzeitig kann die Auflagefläche 142 auf ihrer der Einführspitze 118 zuweisenden Seite in eine Rampe 184 münden, über welche nach dem Ablösen der subkutanen Vorrichtung 122 von der Insertionshilfe 112 die subkutane Vorrichtung 122 gleiten kann, um im Körpergewebe 124 zu verbleiben, während die Insertionshilfe 112 aus dem Körpergewebe 124 herausgezogen wird. Die Ausgestaltungen gemäß den Figuren 3A bis 3C und/oder gemäß Figur 4 können grundsätzlich mit beliebigen anderen Ausführungsbeispielen der vorliegenden Beschreibung, beispielsweise gemäß den Figuren 1A und 1B, 2A und 2B oder 5, kombiniert werden.

Alternativ oder zusätzlich zu dem elektrostatischen Halteprinzip gemäß den Figuren 1A und 1B und/oder dem magnetischen Halteprinzip gemäß den Figuren 2A und 2B lassen sich andere Prinzipien zur Erzeugung einer veränderbaren Haltekraft $F_H$ einsetzen. Ein weiteres Prinzip ist in Figur 5 in einer Ausführungsform dargestellt. Bei diesem Prinzip wird gezielt und über elektrische Einwirkung Einfluss genommen auf Adhäsionskräfte, welche von einer Haftoberfläche 186 ausgeübt werden.

So zeigt beispielsweise Figur 5 schematisch wiederum eine Insertionsvorrichtung 110, wobei weitgehend bezüglich der möglichen Ausgestaltungen auf die obige Beschreibung verwiesen werden kann. Diese umfasst wiederum eine Insertionshilfe 112, beispielsweise mit einem Grundkörper 114 in Form einer Insertionsnadel 116, welche hier lediglich schematisch angedeutet ist. Diese weist eine Auflagefläche 142 auf, auf welcher durch Adhäsionskräfte ein Träger 128 (Sensorsubstrat) der subkutanen Vorrichtung 122 aufliegt, beispielsweise eines subkutanen Sensors 126. Die Haftoberfläche 186 ist in diesem Ausführungsbeispiel Bestandteil der subkutanen Vorrichtung 122. Auch andere Ausgestaltungen sind jedoch möglich, also Ausgestaltungen, bei welchen die Haftoberfläche 186 Bestandteil der Insertionshilfe 112 ist, beispielsweise identisch ist mit der Auflagefläche 142, und/oder Ausgestaltungen, bei welchen sowohl die Insertionshilfe 112 als auch die subkutane Vorrichtung 122 mit zusammenwirkenden Haftoberflächen 186 ausgestattet sind.

In dem dargestellten Ausführungsbeispiel weist der Träger 128 zumindest im Bereich der Haftoberfläche 186 ein elektrisch leitendes oder halbleitendes Polymer auf, welches hier symbolisch angedeutet ist und mit der Bezugsziffer 188 bezeichnet ist. Dieses elektrisch leitende oder halbleitende Polymer kann beispielsweise ganz oder teilweise als konjugiertes Polymer ausgestaltet sein, also als Polymer, bei welchem sich Doppelbindungen und Einfachbindungen abwechseln. Hierdurch entsteht ein ausgedehntes π-Elektronensystem, welches für die leitenden oder halbleitenden Eigenschaften verantwortlich ist. Als Modellsubstanz für ein derartiges konjugiertes Polymer kann Polyacetylen dienen. Alternativ oder zusätzlich sind jedoch auch andere konjugierte Polymere und/oder Oligomere und/oder kleine Moleküle einsetzbar, welche vorzugsweise ebenfalls ein ausgedehntes π-Elektronensystem aufweisen, beispielsweise Polythiophene, Polyparaphenylenvinylen, Polyparaphenylen, Pentacen, Tetracen oder ähnliche Materialien, die dem Fachmann aus dem Bereich der organischen Elektronik grundsätzlich bekannt sind.

In dem vorgeschlagenen Ausführungsbeispiel weist die Insertionsvorrichtung 110 wiederum eine Steuerung 144 auf, über welche gezielt Einfluss auf die Adhäsionseigenschaften der Haftoberfläche 186 genommen werden kann. Zu diesem Zweck kann eine Ladungsträgerdichte und/oder eine Leitfähigkeit der Haftoberfläche 186 gezielt beeinflusst werden. Dies erfolgt im vorliegenden Ausführungsbeispiel über einen Feldeffekt, in Analogie zu einem organischen Feldeffekttransistor. Die Steuerung 144 weist eine erste Spannungsquelle 190 und eine zweite Spannungsquelle 192 auf. Während die erste Spannungsquelle 190 gegenüberliegende Elektroden 194, 196 mit einer Spannung und/oder einem Strom beaufschlagt, welche durch das organische, leitende oder halbleitende Polymer 188 verbunden sind, beaufschlagt die zweite Spannungsquelle 192 eine Gate-Elektrode 198 mit einer Gate-Spannung. Zwischen der Gate-Elektrode 198 und dem Träger 128 kann eine Isolation 200 eingefügt sein. Dementsprechend kann das Potenzial der Gate-Elektrode 198 beeinflusst werden, was wiederum über einen Feldeffekt eine Ladungsträgerdichte und/oder eine Ladungsträgerbeweglichkeit und damit eine Leitfähigkeit im Bereich der Haftoberfläche 186 beeinflusst. Die Leitfähigkeit andererseits beeinflusst wiederum die Haftwirkung, also die Haltekraft zwischen der Insertionshilfe 112 und der subkutanen Vorrichtung 122. Maßgeblich für diese Leitfähigkeit und/oder Ladungsträgerdichte und/oder Beweglichkeit sind, wie oben ausgeführt, insbesondere in den meisten Fällen die π-Elektronen, da die π-Brücken des Polymers 188 bewegliche Ladungen liefern. Durch geeignete Modifikation des Polymers 188 können die Hafteigenschaften gezielt angepasst werden. Im Bereich der Haftoberfläche 186 kann zwischen der Insertionshilfe 112 und der subkutanen Vorrichtung 122 weiterhin mindestens ein Haftvermittler 202 eingefügt sein, beispielsweise ein Klebstoff. Dieser ist in Figur 5 symbolisch mit der Bezugsziffer 202 bezeichnet. Dieser Haftvermittler 202 kann beispielsweise auf der Auflagefläche 142 aufgebracht sein. Die Adhäsion mit diesem Haftvermittler 202 kann beispielsweise gezielt durch die Veränderung der Ladungsträgerdichte und/oder der Leitfähigkeit der Haftoberfläche 186 beeinflusst werden.

Durch Beeinflussung der Spannungen an den Spannungsquellen 190, 192 und/oder der durch diese Spannungsquellen 190, 192 bereitgestellten Ströme, kann somit gezielt Einfluss auf die Haftungseigenschaften und damit auf die Haltekraft genommen werden. Auch so kann mittels der Steuerung 144 die Haltekraft zwischen der subkutanen Vorrichtung 122 und der Insertionshilfe 112 gezielt beeinflusst werden.

Eine weitere prinzipielle Möglichkeit zur Erhöhung einer Haftung zwischen einer subkutanen Vorrichtung 122 und einer Insertionshilfe 112, welche alternativ oder zusätzlich zu den oben beschriebenen Möglichkeiten eingesetzt werden kann, ist die Beeinflussung der Haftung oder die Spaltung von Teilen der Insertionsvorrichtung 110 durch gesteuerte, äußere Einflüsse. So ist es z.B. denkbar, dass gestreckte, unter Vorspannung stehende Folien durch gezielte Krafteinwirkung zerteilt werden, beispielsweise durch Krafteinwirkung auf ein äußeres Ende einer Folie des Trägers 128, wodurch diese Folie reißt oder gespalten wird. Dies kann beispielsweise zu einer schlagartigen Haftungsänderung zwischen der Insertionshilfe 112 und der subkutanen Vorrichtung 122 und/oder von Teilen der subkutanen Vorrichtung 122 führen.

Bezugszeichenliste

| 110 | Insertionsvorrichtung |
| 112 | Insertionshilfe |
| 114 | Grundkörper |
| 116 | Insertionsnadel |
| 118 | Einführspitze |
| 120 | Längserstreckungsachse |
| 122 | Subkutane Vorrichtung |
| 124 | Körpergewebe |
| 126 | Subkutaner Sensor |
| 128 | Träger |
| 130 | Sensorelektroden |
| 132 | Längserstreckungsachse |
| 134 | Implantierbarer Teil |
| 136 | Nicht-implantierbarer Teil |
| 138 | Halter |
| 140 | Sensorkontakte |
| 142 | Auflagefläche |

| | |
|---|---|
| 144 | Steuerung |
| 146 | Erste Elektrode |
| 148 | Erstes Halteelement |
| 150 | Zweite Elektrode |
| 152 | Zweites Halteelement |
| 154 | Isolator |
| 156 | Feldplattenkontakt |
| 158 | Umschaltvorrichtung |
| 160 | Spannungsquelle |
| 162 | Kurzschlussverbindung |
| 164 | Einstechvorrichtung/Ausziehvorrichtung |
| 166 | Permanentmagnet |
| 168 | Magnetischer Pol |
| 170 | Magnetischer Gegenpol |
| 172 | Elektromagnetisches Halteelement |
| 174 | Leiterbahnschleife |
| 176 | Stromquelle |
| 178 | Substrat-Ankerblech |
| 180 | Luftspalt |
| 182 | Stirnfläche |
| 184 | Rampe |
| 186 | Haftoberfläche |
| 188 | Polymer |
| 190 | Erste Spannungsquelle |
| 192 | Zweite Spannungsquelle |
| 194 | Elektrode |
| 196 | Elektrode |
| 198 | Gate-Elektrode |
| 200 | Isolation |

202     Haftvermittler

204     magnetische Feldlinien (Permanentmagnet)

206     magnetische Feldlinien (Leiterbahnschleife)


**Patentansprüche**

1.  Insertionsvorrichtung (110) zur zumindest teilweisen Insertion einer subkutanen Vorrichtung (122) in ein Körpergewebe (124), insbesondere eines subkutanen Sensors (126) zur Erfassung mindestens eines Analyten, wobei die Insertionsvorrichtung (110) mindestens eine Insertionshilfe (112) und mindestens eine subkutane Vorrichtung (122) aufweist, wobei die Insertionshilfe (112) mindestens einen im Wesentlichen starr ausgestalteten Grundkörper (114) zum Einführen in das Körpergewebe (124) aufweist, insbesondere eine Insertionsnadel (116), wobei die Insertionsvorrichtung (110) eingerichtet ist, um zwischen dem Grundkörper (114) und der subkutanen Vorrichtung (122) eine veränderbare Haltekraft zu erzeugen, wobei die Insertionsvorrichtung (110) eingerichtet ist, um während des Einführens die Haltekraft derart einzustellen, dass die subkutane Vorrichtung (122) an dem Grundkörper (114) gehalten wird und wobei die Insertionsvorrichtung (110) weiterhin eingerichtet ist, um nach dem Einführen die Haltekraft derart einzustellen, dass die subkutane Vorrichtung (122) von dem Grundkörper (114) lösbar ist.

2.  Insertionsvorrichtung (110) nach dem vorhergehenden Anspruch, wobei die Haltekraft eingerichtet ist, um während des Einführens eine räumliche Verlagerung zwischen der subkutanen Vorrichtung (122) und dem Grundkörper (114) in einer Insertionsrichtung zumindest weitgehend zu verhindern.

3.  Insertionsvorrichtung (110) nach einem der vorhergehenden Ansprüche, wobei die Haltekraft eine nicht-mechanische Haltekraft umfasst.

4.  Insertionsvorrichtung (110) nach einem der vorhergehenden Ansprüche, wobei die Insertionsvorrichtung (110) eingerichtet ist, um die Haltekraft nach dem Einführen derart einzustellen, dass die subkutane Vorrichtung (122) von dem Grundkörper (114) abgestoßen wird.

5.  Insertionsvorrichtung (110) nach einem der vorhergehenden Ansprüche, wobei die Haltekraft eine elektrostatische Haltekraft umfasst.

6.  Insertionsvorrichtung (110) nach dem vorhergehenden Anspruch, wobei der Grundkörper (114) mindestens eine erste Elektrode (146) aufweist, wobei die subkutane Vorrichtung (122) mindestens eine zweite Elektrode (150) aufweist, wobei die Insertionsvorrichtung (110) eingerichtet ist, um die erste Elektrode (146) und die zweite Elektrode (150) mit unterschiedlichen elektrischen Potenzialen zu beaufschlagen.

7.  Insertionsvorrichtung (110) nach dem vorhergehenden Anspruch, wobei zwischen der ersten Elektrode (146) und der zweiten Elektrode (150) mindestens ein Isolator (154) angeordnet ist.

8.  Insertionsvorrichtung (110) nach einem der vorhergehenden Ansprüche, wobei die Haltekraft eine magnetische Haltekraft umfasst.

9.  Insertionsvorrichtung (110) nach dem vorhergehenden Anspruch, wobei die Insertionshilfe (112) und/oder die subkutane Vorrichtung (122) mindestens ein elektromagnetisches Halteelement (172) zur Erzeugung eines magnetischen Feldes, insbesondere mindestens eine Leiterbahnschleife (174), umfasst.

10. Insertionsvorrichtung (110) nach dem vorhergehenden Anspruch, wobei die Insertionsvorrichtung (110) weiterhin mindestens eine Stromquelle (176) zur Erzeugung eines veränderbaren Stroms aufweist.

11. Insertionsvorrichtung (110) nach einem der vorhergehenden Ansprüche, wobei die Insertionsvorrichtung (110) eingerichtet ist, um eine elektrische Ladungsträgerdichte und/oder eine elektrische Leitfähigkeit im Bereich mindestens einer Haftoberfläche (186) der Insertionshilfe (112) und/oder der subkutanen Vorrichtung (122) zu beeinflussen.

12. Insertionsvorrichtung (110) nach dem vorhergehenden Anspruch, wobei die Beeinflussung durch einen Feldeffekt erfolgt.

**13.** Insertionsvorrichtung (110) nach einem der beiden vorhergehenden Ansprüche, wobei die Haftoberfläche (186) mindestens ein leitfähiges oder halbleitendes organisches Material aufweist, insbesondere ein konjugiertes Polymer (188).

**14.** Insertionsvorrichtung (110) nach einem der vorhergehenden Ansprüche, wobei die Insertionhilfe (112) eine Auflagefläche (142) zum Anlegen der subkutanen Vorrichtung (122) während des Einführens aufweist, insbesondere eine im Wesentlichen ebene Auflagefläche (142).

**15.** Insertionshilfe (112) zur zumindest teilweisen Insertion einer subkutanen Vorrichtung (122) in ein Körpergewebe (124), insbesondere zum Einsatz in einer Insertionsvorrichtung (110) nach einem der vorhergehenden, eine Insertionsvorrichtung (110) betreffenden Ansprüche, wobei die Insertionshilfe (112) mindestens einen im Wesentlichen starr ausgestalteten Grundkörper (114) zum Einführen in das Körpergewebe (124) aufweist, insbesondere eine Insertionsnadel (116), wobei die Insertionshilfe (112) eingerichtet ist, um mit der subkutanen Vorrichtung (122) derart zusammenzuwirken, dass zwischen dem Grundkörper (114) und der subkutanen Vorrichtung (122) eine veränderbare Haltekraft erzeugbar ist.

**16.** Subkutane Vorrichtung (122) zur Insertion in ein Körpergewebe (124), insbesondere subkutaner Sensor (126) zur Erfassung mindestens eines Analyten, insbesondere zum Einsatz in einer Insertionsvorrichtung (110) nach einem der vorhergehenden, eine Insertionsvorrichtung (110) betreffenden Ansprüche, wobei die subkutane Vorrichtung (122) eingerichtet ist, um mit einem Grundkörper (114) einer Insertionshilfe (112) zum Einführen in das Körpergewebe (124) derart zusammenzuwirken, dass zwischen dem Grundkörper (114) und der subkutanen Vorrichtung (122) eine veränderbare Haltekraft erzeugbar ist.

Fig. 1 A

Fig. 1 B

Fig. 2 A

110

206    174    122, 126    174    206

128

204    204

112, 114, 116

180  178    168   148, 166    142
170

**Fig. 2 B**

110    110    110

122, 126    122, 126    122, 126

142

112, 114, 116    112, 114, 116    142    112, 114, 116    142

**Fig. 3 A**    **Fig. 3 B**    **Fig. 3 C**

Fig. 4

Fig. 5

Europäisches
Patentamt

European
Patent Office

Office européen
des brevets

**EUROPÄISCHER RECHERCHENBERICHT**

Nummer der Anmeldung

EP 09 16 0090

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/156126 A1 (FLAHERTY J C [US]) 5. Juli 2007 (2007-07-05) * Absätze [0021], [0085], [0086], [0089], [0123] - [0125] * ----- | 1-3,8,9, 14-16 | INV. A61B5/00 A61M5/158 |
| D,A | WO 03/080169 A (NOVO NORDISK AS [DK]) 2. Oktober 2003 (2003-10-02) * Seite 20, Zeilen 10-30 * * Seite 22, Zeilen 10-29; Abbildung 5a * ----- | 1-16 | |
| | | | **RECHERCHIERTE SACHGEBIETE (IPC)** |
| | | | A61B A61M |

Der vorliegende Recherchenbericht wurde für alle Patentansprüche erstellt

| Recherchenort | Abschlußdatum der Recherche | Prüfer |
|---|---|---|
| München | 13. Oktober 2009 | Manschot, Jan |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 16 0090

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-10-2009

| Im Recherchenbericht angeführtes Patentdokument | Datum der Veröffentlichung | Mitglied(er) der Patentfamilie | Datum der Veröffentlichung |
|---|---|---|---|
| US 2007156126    A1 | 05-07-2007 | KEINE | |
| WO 03080169    A | 02-10-2003 | AT        424231 T<br>AU    2003226902 A1<br>EP       1490142 A1<br>JP    2005520648 T | 15-03-2009<br>08-10-2003<br>29-12-2004<br>14-07-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060015024 A1
- EP 0678308 B1
- DE 10306013 A1
- US 20060030824 A
- DE 102004002472 B4
- US 20070016149 A

- DE 10117286 A1
- US 20040158230 A
- US 20080033399 A
- WO 03080169 A1
- WO 03080169 A